(19) Europäisches Patentamt
European Patent Office
Office européen des brevets

(11) Publication number: 0 266 890 A1

# (12) EUROPEAN PATENT APPLICATION

(21) Application number: 87308663.1

(22) Date of filing: 30.09.87

(51) Int. Cl.⁴: C07D 471/04 , A61K 31/435 , C07D 213/76 , //(C07D471/04,235:00,221:00)

(30) Priority: 07.10.86 JP 239863/86

(43) Date of publication of application: 11.05.88 Bulletin 88/19

(84) Designated Contracting States: AT BE CH DE ES FR GB GR IT LI LU NL SE

(71) Applicant: YAMANOUCHI PHARMACEUTICAL CO. LTD.
No. 3-11 Nihonbashi-Honcho, 2-chome
Chuo-ku
Tokyo(JP)

(72) Inventor: Yanagisawa, Isao
22-8, Shakujiidai 2-chome Nerima-ku
Tokyo(JP)
Inventor: Ohta, Mitsuaki
8-20, Minami 1-chome
Wako-shi Saitama(JP)
Inventor: Koide, Tokuo
16-1, Hasune 3-chome Itabashi-ku
Tokyo 174(JP)
Inventor: Shikama, Hisataka
Kaga Garden Hatsu 420 3-1, Kaga 2-chome
Itabashi-ku Tokyo 173(JP)
Inventor: Miyata, Keiji
28-2-102, Nishi Ochiai 1-chome Shinjuku-ku
Tokyo 161(JP)

(74) Representative: Geering, Keith Edwin et al
REDDIE & GROSE 16 Theobalds Road
London WC1X 8PL(GB)

## (54) Imidazopyridine derivatives, their production, and pharmaceutical compositions containing them.

(57) The present invention relates to imidazopyridine derivatives of formula (I) below(and pharmaceutically acceptable salts thereof) which possess $H^+$, $K^+$-adenosine triphosphatase inhibition activity and are useful as prophylactic and treating agents for gastric diseases; to pharmaceutical compositions containing them; and to processes for production thereof

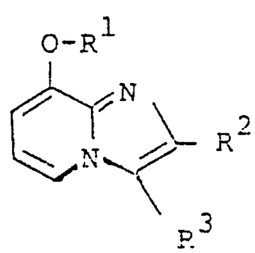

(I)

EP 0 266 890 A1

wherein:

$R^1$ represents an alkenyl group having 4 to 6 carbon atoms,or an alkyl group having 1 to 10 carbon atoms which may have a cycloalkyl group substituent;

$R^2$ represents a hydroxy group, a lower alkoxycarbonyl group, a lower alkyl group which may have a hydroxy, lower alkoxy, or lower alkoxycarbonyl group substituent, a phenyl group which may have a lower alkoxy group substituent or a lower acyloxyalkyl group which may have a lower acyloxy group substituent; and,

$R^3$ represents a hydrogen atom, a lower alkyl group, a halogeno-lower alkyl group, a lower alkoxyalkyl group, a hydroxy-lower alkyl group, a cyano-lower alkyl group, a mono-or di-lower alkylamino-lower alkyl group, an amino group, a mono-or di-lower alkylamino group, a nitroso group, a lower alkoxy-carbonyl group, a lower acylamino group, a lower alkylsulfonylamino group, a group of formula:

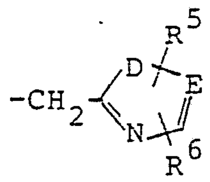

(wherein D is NH or S, E is N or CH,and $R^5$ and $R^6$ are the same or different and selected from a hydrogen atom and lower alkyl, lower alkoxycarbonyl and phenyl groups); a group of formula:

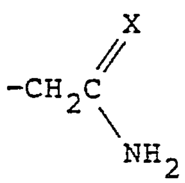

(wherein X is O, S or N-$R^7$ wherein $R^7$ is a sulfamoyl group, a lower acylamino group or a lower alkynyl group); or a group of formula:

$-CH_2-Y-R^8$

(wherein Y is O or S and $R^8$ is a cyano-lower alkyl group or a lower alkynyl group).

# IMIDAZOPYRIDINE DERIVATIVES, THEIR PRODUCTION, AND PHARMACEUTICAL COMPOSITIONS CONTAINING THEM.

The present invention relates to imidazopyridine derivatives of formula (I) below(and pharmaceutically acceptable salts thereof) which possess $H^+$,$K^+$-adenosine triphosphatase inhibition activity and are useful as prophylactic and treating agents for gastric diseases; to pharmaceutical compositions containing them; and to processes for production thereof

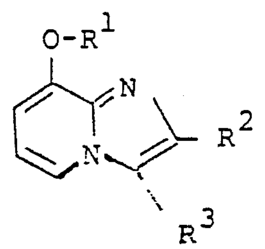

(I)

wherein:

$R^1$ represents an alkenyl group having 4 to 6 carbon atoms,or an alkyl group having 1 to 10 carbon atoms which may have a cycloalkyl group substituent:

$R^1$ represents an alkenyl group having 4 to 6 carbon atoms, or an alkyl group having 1 to 10 carbon atoms which may have a cycloalkyl group substituent;

$R^2$ represents a hydroxy group, a lower alkoxycarbonyl group, a lower alkyl group which may have a hydroxy, lower alkoxy, or lower alkoxycarbonyl group substituent, a phenyl group which may have a lower alkoxy group substituent or a lower acyloxyalkyl group which may have a lower acyloxy group substituent; and,

$R^3$ represents a hydrogen atom, a lower alkyl group, a halogeno-lower alkyl group, a lower alkoxyalkyl group, a hydroxy-lower alkyl group, a cyano-lower alkyl group, a mono-or di-lower alkylamino-lower alkyl group, an amino group, a mono-or di-lower alkylamino group, a nitroso group, a lower alkoxy-carbonyl group, a lower acylamino group, a lower alkylsulfonylamino group, a group of formula:

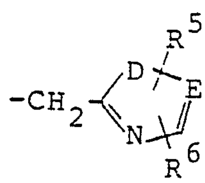

(wherein D is NH or S, E is N or CH,and $R^5$ and $R^6$ are the same or different and selected from a hydrogen atom and lower alkyl, lower alkoxycarbonyl and phenyl groups); a group of formula

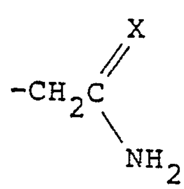

(wherein X is O, S or N-$R^7$ wherein $R^7$ is a sulfamoyl group, a lower acylamino group or a lower alkynyl group); or a group of formula:

$-CH_2-Y-R^8$

(wherein Y is O or S and $R^8$ is a cyano-lower alkyl group or a lower alkynyl group).

8-Aralkyl (or aryl)oxyimidazo[1,2-a]pyridines which optionally have a substituent(s) on the phenyl group are disclosed in European Patent Nos. 33094 and 68378. It is described there that these pyridines are useful as anti-secretion agents and cell protecting agents.

The compounds of the present invention are novel compounds which have different substituents from these known compounds and have excellent $H^+,K^+$-adenosine triphosphatase inhibitory activity.

In this specification, the term "lower" refers to a straight or branched carbon chain having up to 6 carbon atoms, unless otherwise indicated. Accordingly, a lower alkyl group means a straight or branched alkyl group having 1 to 6 carbon atoms. Concrete examples of the lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, 1-ethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl groups,etc.

As lower alkoxy groups, mention may be made of straight or branched alkoxy groups having 1 to 6 carbon atoms, for example, methoxy, ethoxy, propoxy, isopropoxy, butoxy, isobutoxy, sec-butoxy, tert-butoxy, pentyloxy, isopentyloxy, hexyloxy and isohexyloxy groups and the like.

A lower alkynyl group is a straight or branched alkynyl group having 2 to 6 carbon atoms and is exemplified by ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 3-methyl-1-butynyl, 2-methyl-3-butynyl, 1-methyl-2-butynyl, 1-methyl-3-butynyl, 1,1-dimethyl-2-propynyl, 1-hexynyl, 2-hexynyl, 3-hexynyl, 4-hexynyl and 5-hexynyl groups and the like.

As the alkyl group $R^1$, a straight or branched alkyl group having 1 to 10 carbon atoms is preferred. Concrete examples of the alkyl group include, in addition to the concrete examples of the lower alkyl group described above, heptyl, 5-methylhexyl, 1-methylhexyl, 3-ethylpentyl, 2-propylbutyl, octyl, 6-methylheptyl, nonyl, 7-methyloctyl, decyl and 8-methylnonyl groups and the like.

As alkenyl groups having 4 to 6 carbon atoms, mention may be made (as preferred examples) of 1-butenyl, 2-butenyl, 3-butenyl, 2-methyl-1-propenyl, 2-methyl-2-propenyl, 1-pentenyl, 2-pentenyl, 3-pentenyl, 4-pentenyl, 3-methyl-1-butenyl, 3-methyl-2-butenyl, 3-methyl-3-butenyl, 2,2-dimethyl-2-propenyl, 1-hexenyl, 2-hexenyl, 3-hexenyl, 4-hexenyl, 5-hexenyl, 4-methyl-1-pentenyl, 4-methyl-2-pentenyl, 4-methyl-3-pentenyl, 4-methyl-4-pentenyl and 3-methyl-2-pentenyl groups and the like.

As the cycloalkyl group, preferred are groups having 3 to 6 carbon atoms. Concrete examples include cyclopropyl, cyclobutyl, cyclopentyl and cyclohexyl groups etc.

As the acyl group of acyloxy or acyl-lower alkyl groups,preferred are a lower alkanoyl group and an arylcarbonyl group, more preferably, a straight or branched alkanoyl group having 2 to 6 carbon atoms or a benzoyl group.
Specific examples of the alkanoyl group include acetyl, propionyl, butryryl, isobutyryl, valeryl, isovaleryl, pivaloyl and hexanoyl groups and the like.

As the alkyl group having a lower alkoxy or a cycloalkyl group substituent, mention may be made of groups in which an optional hydrogen atom in the alkyl group described above is substituted with a lower alkoxy group or cycloalkyl group described above; representative examples of the lower alkoxy-substituted alkyl group include methoxymethyl, methoxyethyl, methoxypropyl, methoxybutyl, methoxypentyl, ethoxymethyl, ethoxyethyl, ethoxypropyl, ethoxybutyl, ethoxypentyl, propoxymethyl, propoxyethyl, propoxypropyl, propoxybutyl, propoxypentyl, isopropoxymethyl, isopropoxyethyl, isopropoxypropyl, isopropoxybutyl, isopropoxypentyl, isopropoxyhexyl, butoxymethyl, butoxyethyl, butoxypropyl, butoxybutyl, isobutoxymethyl, isobutoxyethyl, isobutoxypropyl, and isobtuoxybutyl groups and the like; specific examples of the cycloalkyl-substituted alkyl group include cyclopropylmethyl, cyclopropylethyl, cyclopropylpropyl, cyclopropylbutyl, cyclobutylmethyl, cyclobutylethyl, cyclobutylpropylcyclopentylmethyl, cyclopentylethyl, cyclopentylpropyl, cyclohexylmethyl, cyclohexylethyl, cyclohexylpropyl, cyclohexylbutyl, cyclohexylpentyl and cyclohexylhexyl groups and the like.

A lower alkoxy-substituted phenyl group is a group in which the hydrogen atom at the ortho-, para-or meta-positions of the phenyl group is substituted with a lower alkoxy group described above; particularly preferred are ortho-or para-methoxphenyl, ethoxyphenyl, propoxyphenyl and isopropoxyphenyl groups.

As the halogen atom, particularly preferred are fluorine, chlorine bromine atoms.

Thus, a halogeno-substituted lower alkyl group is a group in which optional hydrogen atom(s) (e.g. 1 to 3) in the lower alkyl group described above is/are substituted with the aforesaid halogen atom(s). Taking a chlorine atom as an example, there are chloromethyl, dichloromethyl, trichloromethyl, 1-chloroethyl, 2-chloroethyl, 1-chloropropyl, 2-chloropropyl, 3-chloropropyl, 2-chloro-2-methylethyl, 1-chlorobutyl, 2-chlorobutyl, 3-chlorobutyl, 4-chlorobutyl, 5-chloropentyl and 5-chlorohexyl groups and the like.

A hydroxy-lower alkyl group is a group in which an optional hydrogen atom of the lower alkyl group described above is substituted with a hydroxy group. Concrete examples are straight or branched hydroxyalkyl groups having 1 to 6 carbon atoms such as hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, 1-hydroxybutyl, 2-hydroxybutyl, 3-hydroxybutyl, 4-hydroxybutyl, 1-hydroxy-2-methylpropyl, 2-hydroxy-2-methylpropyl, 3-hydroxy-2-methylpropyl, 1-hydroxypentyl, 2-hydroxypentyl, 3-hydroxypentyl, 4-hydroxpentyl, 5-hydroxypentyl, 4-hydroxyhexyl and 5-hydroxy-4-methylpentyl groups and the like.

Concrete examples of the cyano-substituted lower alkyl group include cyanomethyl, 1-cyanoethyl, 2-cyanoethyl, 1-cyanopropyl, 2-cyanopropyl, 3-cyanopropyl, 1-cyano-1-methylethyl, 2-cyano-1-methylethyl, 1-cyanobutyl, 2-cyanobutyl, 3-cyanobutyl, 4-cyanobutyl, 1-cyano-1-methylpropyl, 2-cyano-1-methylpropyl, 3-cyano-1-methylpropyl, 1-cyano-2-methylpropyl, 2-cyano-2-methypropyl, 3-cyano-2-methylpropyl, 1-cyanomethylpropyl, 1-cyanopentyl, 2-cyanopentyl, 3-cyanopentyl, 4-cyanopentyl, 5-cyanopentyl, 1-cyano-3-methylbutyl, 2-cyano-3-methylbutyl, 1-cyanomethylbutyl, 1-cyanohexyl, 2-cyanohexyl, 3-cyanohexyl, 4-cyanohexyl, 5-cyanohexyl and 6-cyanohexyl groups and the like.

A mono-or di-lower alkylamino group is a group in which one or two hydrogen atoms of an amino group is/are substituted with lower alkyl group(s) described above. Concrete examples include mono-alkylamino groups with a straight or branched alkyl group having 1 to 6 carbon atoms such as methylamino, ethylamino, propylamino, isopropylamino, butylamino, isobutylamino, pentylamino, isopentylamino and hexylamino groups and the like; symmetric di-alkylamino groups with straight or branched alkyl groups have 1 to 6 carbon atoms such as dimethylamino, diethylamino, dipropylamino, diisopropylamino, dibutylamino, dipentylamino and dihexylamino groups; and asymmetric di-alkylamino groups with different straight or branched alkyl groups having 1 to 6 carbon atoms such as ethylmethylamino, methypropylamino, ethylpropylamino, butylmethylamino, butylethylamino and butylpropylamino groups and the like.

A mono-or di-lower alkylamino-substituted lower alkyl group is a group in which optional hydrogen atom(s) of a lower alkyl group described above is/are susbstituted with mono-or di-lower alkylamino group-(s) described above. Taking the diethylamino group as an example, specific examples are diethylaminomethyl, 2-(N,N-diethylamino)ethyl, 3-(N,N-diethylamino)propyl, 4-(N,N-diethylamino)butyl, 5-(N,N-diethylamino)pentyl and 6-(N,N-diethylamino)hexyl groups and the like; however, the groups are not particularly limited to those substituted with the diethylamino group.

As lower alkoxycarbonyl groups,mention may be made of monovalent groups given by ester formation between a straight or branched alcohol having 1 to 6 carbon atoms and a carboxy compound, such as methoxycarbonyl, propoxycarbonyl, isopropoxycarbonyl, butoxycarbonyl, isobutoxycarbonyl, sec-butoxycarbonyl, pentyloxycarbonyl, isopentyloxycarbonyl, neopentyloxycarbonyl, tert-pentyloxycarbonyl and hexyloxycarbonyl groups and the like.

Some compounds of the present invention have stereoisomers such as optical isomers, tautomers and the like.

The present invention includes individual isomers and mixtures thereof.

the compounds (I) may also form salts. The present invention includes the salts of compounds (I), in particular, pharmaceutically acceptable salts thereof.

Examples of such salts include acid addition salts with inorganic acids such as hydrochloric, hydrobromic, sulfuric, nitric and phosphoric acids or the like;and with organic acids such as formic, acetic, oxalic, citric, succinic, fumaric, maleic, tartaric, methanesulfonic and ethanesulfonic acids or the like. Depending upon the substituents present, there may also be salts with bases such as sodium, potassium, etc. and quaternary ammonium salts with halogenated lower alkyl groups.

The compounds of the present invention can be prepared by various processes. Representative examples of the processes will be given below. All processes according to the invention optionally include the step of converting the initial product to or from salt form.

Process 1:

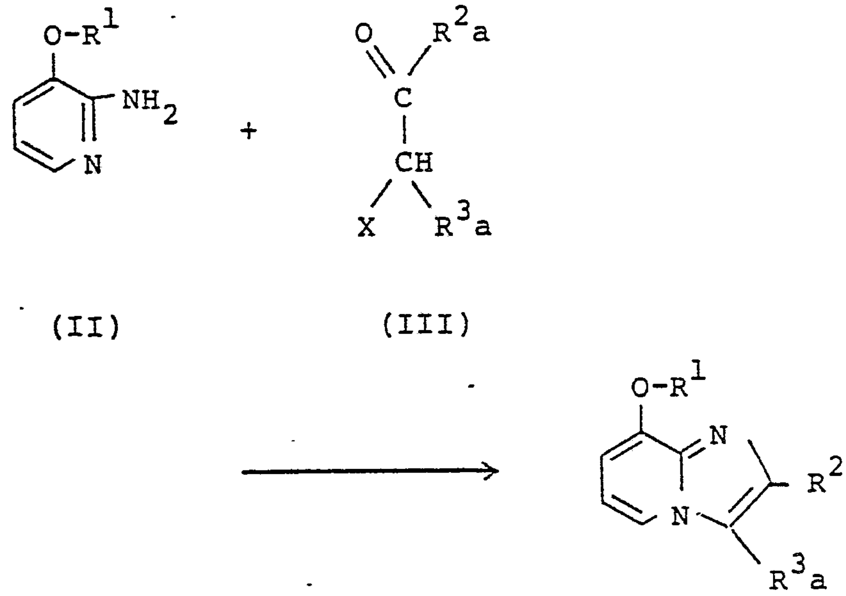

(II) (III)

(Ia)

wherein $R^1$ and $R^2$ have the same meanings as above; $R^2a$ represents a halogen atom, a lower alkoxycarbonyl group, a lower alkyl group which may have a lower alkoxycarbonyl group substituent or a phenyl group which may have a lower alkoxy group substituent; $R^3a$ represents a hydrogen atom, a lower alkyl group, a cyano-lower alkyl group or a lower alkoxycarbonyl group; and X represents a halogen atom or an organic sulfonic acid residue.

The compounds represented by general formula (Ia) can be produced by reacting amino-substituted pyridyl ethers represented by general formula (II) with compounds represented by general formula (III).

In the reaction scheme described above, examples of the halogen atom X are iodine, bromine, chlorine, etc; and examples of the organic sulfonic acid residue are alkanesulfonic acid residues such as methanesulfonyloxy and ethanesulfonyloxy groups and the like, and aromatic sulfonic acid residues such as benzenesulfonyloxy and toluenesulfonyloxy groups and the like.

The reaction can be advantageously carried out using the compounds (II) and (III) either in almost equimolar amounts or with a slight excess of (II) or (III), in an inert organic solvent, preferably in the presence of a base, at room temperature or with heating, preferably with heating to reflux. As the inert organic solvent ether, benzene, toluene, xylene or the like can be advantageously employed when $R^2a$ is a halogen atom; when $R^2a$ is other than a halogen atom, an alcohol such as methanol, ethanol, isopropanol or the like, benzene, toluene, xylene or the like can be advantageously employed.

When a base is employed, it is preferred to use trimethylamine, triethylamine, pyridine, picoline, lutidine, dimethylaniline, potassium carbonate, sodium carbonate, sodium hydrogencarbonate, etc.

Process 2:

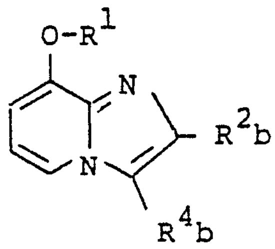

(IV)

or

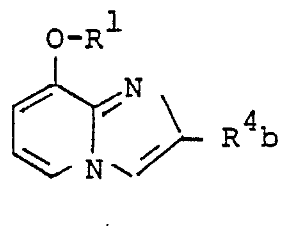

(IV')

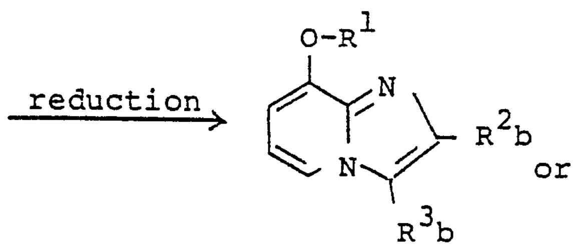


(Ib)

or

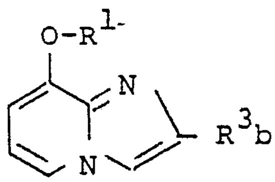

(I'b)

wherein $R^1$ has the same meaning as above; $R^2b$ is a lower alkyl group; $R^4b$ represents a group that can be converted into a hydroxy-lower alkyl group or an amino group upon reduction; and $R^3b$ represents a hydroxy-lower alkyl group, a cyano-lower alkyl group or an amino group.

The compounds of the present invention represented by general formula (Ib) or (I'b) can be produced by reducing the compounds represented by general fromula (IV) or IV').

As the group that can be converted into a hydroxy-lower alkyl group upon reduction mention may be made of a lower alkoxycarbonyl-lower alkyl group ($C_3$ to $C_{12}$) or a hydroxy-lower alkyl group wherein the hydroxy group has been protected. As the protective group of this hydroxy group, mention may be made of benzyl, p-methoxybenzyl and benzyloxycarbonyl groups, protective groups of the acetal type, acetyl and benzoyl groups and the like.

As the group that can be converted into a cyano-lower alkyl group upon reduction, mention may be made of ($C_1$-$C_6$ alkyl)$CH_2NO_2$ groups,

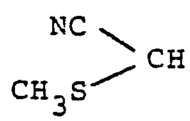 - ($C_1$-$C_6$ alkyl) groups, etc.

As the group that can be converted into an amino group upon reduction, mention may be made of cyano, nitro, nitroso and protective amino groups similar to those described above.

The reduction of these compounds can be by catalytic reduction in the presence of various catalysts, chemical reduction with various hydrides or metals and acids or liquid ammonia and metallic sodium or the like, and reductive dehydration which is effected by phosphorus trichloride in pyridine.

To reduce the protective group of, for example, an hydroxy or amino group to split it off catalytic reduction using palladium-carbon as a catalyst is advantageously applied in conventional manner.

For the reduction of a lower alkoxycarbonyl group into hydroxymethyl group, it is advantageous to react with aluminum lithium hydride in an organic solvent such as ether, dioxane, tetrahydrofuran, etc. The reaction can be appropriately carried out in an inert gas under anhydrous conditions.

To reduce a nitro or nitroso group or the like to convert into an amino group, it is advantageous to conduct catalytic reduction using as a catalyst palladium-carbon, Raney nickel, palladium black, etc. Further for the reduction of a group shown by formula:

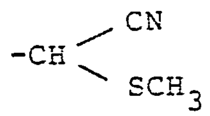

to convert into a cyanomethyl group, catalytic reduction using Raney nickel or the like as a catalyst is preferred. To convert a nitromethyl into a cyano group, it is advantageous to apply thereto reductive dehydration by reaction with phosphorus trichloride in pyridine. Further for the reduction of a cyano group to convert it to an aminomethyl group, chemical reduction using aluminum lithium hydride is suited.

When two or more substituents to be reduced are present, these reductions can be performed by reducing all of the substituents either simultaneously or stepwise by appropriately selecting the conditions; alternatively, one or some only of the substituents can be reduced by suitably choosing the conditions.

Process 3:

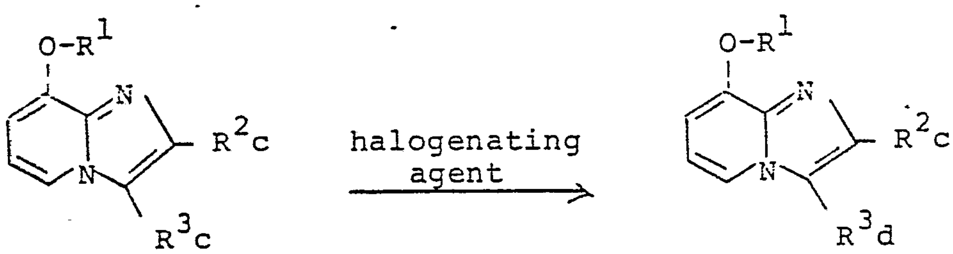


(Ic) (Id)

wherein $R^1$ has the same meaning as above; and $R^2c$ represents a lower alkyl group or a phenyl group which may have a lower alkoxy group substituent; $R^3c$ represents a hydroxy-lower alkyl group; and $R^3d$ represents a halogeno-lower alkyl group.

Among the compounds of the present invention, the imidazopyridien derivatives (Id), at the 3-position of which is a halogeno-lower alkyl group, can be produced by reacting halogenating agent with the corresponding hydroxy-lower alkyl group-containing compounds (Ic).

As the halogenating agent mention may be made of hydrogen halides such as hydrogen fluoride, hydrogen bromide, etc.; thionyl halides such as thionyl chloride or the like; alkali halides such as sodium bromide, etc.; phosphorus halides, phosphorus oxyhalides, triphenyl phosphonate alkali halides, triphenylphosphine carbon tetrahalides, triphenylphosphine dihalogenides, diphenyltrihalogenophosphoranes, triphenyl phosphonate dihalogenides, sulfonyl halogenides, sulfonium halogenides, etc.

The reaction varies depending upon the raw compounds, desired compounds and kind of halogenating agents. When using as the halogenating agent, for example, thionyl halides such as thionyl chloride, thionyl bromide or the like, it is preferred to use primary or secondary alcohol as raw compound. The reaction can be carried out by reacting the compounds (Ic) or salts thereof with the halogenating agents in almost equimolar amount or with an excess amount of either compound, preferably in the absence of any solvent or in an organic solvent such as methylene chloride, ether, dioxane, tetrahydrofuran, etc., under cooling or at room temperature. Depending upon the reaction conditions, on some occasions it may be appropriate to conduct the reaction in the presence of amines such as pyridine, etc.

Process 4:

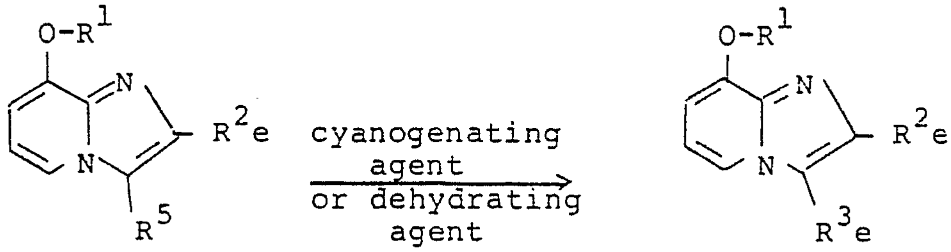


(V) (Ie)

wherein $R^1$ has the same meaning as above; $R^2e$ represents a lower alkyl group which may be substituted with a lower alkoxy group, or a phenyl group which may be substituted with a lower alkoxy group; $R^5$ represents a moiety that can be converted into a cyano-lower alkyl group; and $R^3e$ represents a cyano-lower alkyl group.

Among the compounds of the present invention, the imidazopyridine derivatives (Ie) containing a cyano group can be produced by reacting cyanogenating agents or dehydrating agents with the compounds represented by general formula (V).

As the moiety that can be converted into the cyano-lower alkyl group, atoms or groups corresponding to cyano are a hydrogen atom, a halogen atom, an organic sulfonic acid residue, and trialkylsilyl, lower alkoxy, phenoxy, trialkylammonium, carbamoyl, formyl, lower alkoxycarbonyl, carboxy, aminomethyl, aminocarboxymethyl

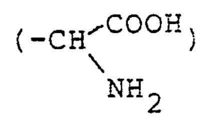

and hydroxyiminomethyl (-CH = N = OH) groups etc.

Methods of converting these moieties into the cyano group include nucleophilic substitution, dehydration, oxidation and the like, in which the cyanogenating agent varies in the respective reactions. For example, in nucleophilic substitution, there can be used alkali cyanides such as sodium cyanide, potassium cyanide and the like; metal cyanides such as silver cyanide, copper cyanide, cyano-coordinated transition metal complexes and the like, cyanic acid, etc. In forming the cyano group through dehydration of an amide or hydroxyiminomethyl group, a dehydrating agent is employed. The aforesaid cyanogenating agent is defined as including such a dehydrating agent. As the dehydrating agent in such a reaction, there can be employed phosphorus pentoxide, phosphorus pentachloride, phosphorus trichloride, phosphorus oxychloride, trifluoracetic anhydride, triphenylphosphine, tris(triphenylphosphine)chlororhodium, phosphonitrilic chloride, thionyl chloride, phosgene, aluminum chloride, dicyclohexylcarbodiimide, a trialkylsilyl chloride, acetyl chloride, benzoyl chloride, sulfonium chloride, polyphosphoric acid, tin oxide, titanium tetrachloride, trichlorotriazine, etc.

The aldehyde and the carboxy are reacted with hydroxylamine or amine to produce the oxime or amide, which is converted to the cyano group by reacting with dehydrating agent described above.

Methods of forming the cyano group by oxidation, include reacting with an aminomethyl compound in the presence of lead tetroxide or nickel peroxide; reacting sodium hypochlorite, a hypobromite or N-bromosuccinimide with an amino acid salt; and reacting with a primary alcohol or aldehyde together with nickel/alumina, cupric chloride or the like in the presence of ammonia. These treating agents are also included in the cyanogenating agent described above.

Reaction conditions vary depending upon the kind of cyanogenating agent, raw compounds and desired compounds. For example, when the moiety corresponding to the cyano group is an atom or group such as a halogen atom, an organic sulfonic acid residue or the like that is split off by substitution, it is advantageous to react the compounds (V) with an equimolar or excess amount of the alkali cyanides, based on the compounds (V), in an inert organic solvent such as dimethylsulfoxide, dimethylformamide, etc. under cooling to room temperature, preferably in an inert gas under anhydrous conditions.

When the moiety corresponding to the cyano group is group such as an amide, an oxime or the like that is converted into the cyano group upon dehydration, it is advantageous to react the compounds (V) with the dehydrating agents described above, in a slight excess over the theoretical amount, in an organic solvent such as benzene, toluene, xylene, mesitylene, pyridine, chloroform, dichloromethane, dichloroethane, etc.

The oxidation can be carried out by reacting the oxidizing agent described above, in an organic solvent such as an alcohol, e.g., methanol, ethanol or the like, benzene, toluene, xylene, dichloromethane, dichloroethane, etc.

Process 5:

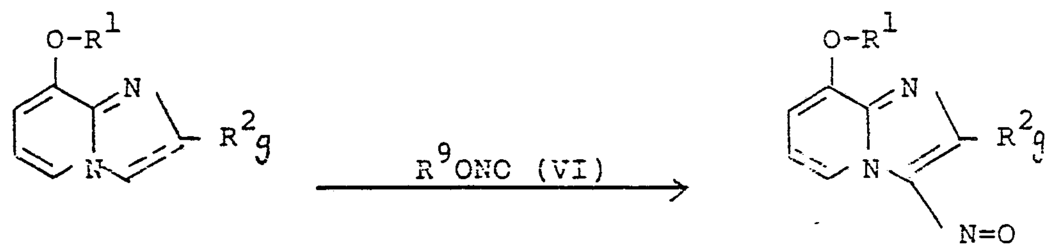


(If) (Ig)

wherein $R^1$ has the same meaning as above; $R^2g$ is a lower alkyl group or a phenyl group which may have a lower alkoxy group substituent; and $R^9$ represents an alkali metal atom, a hydrogen atom or a lower alkyl group.

Among the compounds of the present invention, the compounds containing a nitroso group at the 3-position thereof can be produced by reacting the imidazopyridine derivatives (If),at the 3-position of which is hydrogen, with nitroso agents (VI) such as nitric acid or a nitric acid lower ester, etc.

The reaction is performed by reacting the compounds (If) with the compounds (VI) in an equimolar or excess amount based on (If) either in an organic solvent such as ether, dioxane, tetrahydrofuran, acetic acid or the like or in an aqueous hydrochloric acid solution, at room temperature or with heating.

Process 6:

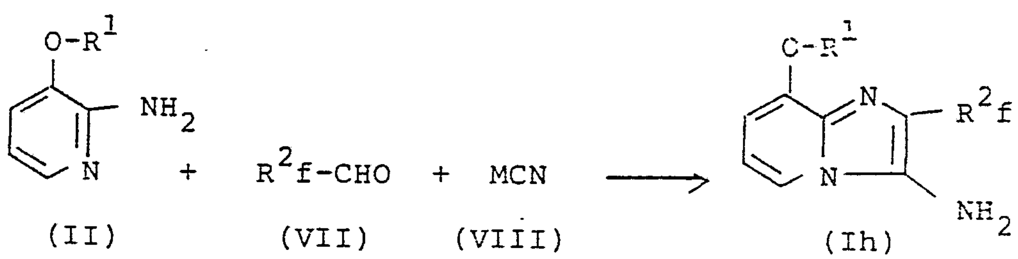


(II) (VII) (VIII) (Ih)

wherein $R^1$ has the same meaning as above; $R^2f$ represents a lower alkyl group; and M represents an alkali metal atom.

The compounds of the present invention represented by general formula (Ih) can be produced by reacting the amino-substituted pyridine ethers represented by general formula (II) with aldehydes (VII) and alkali metal cyanides (VIII).

As the alkali metal atom M in the reaction scheme above, mention may be made of sodium, potassium, lithium, etc.

It is advantageous that the reaction be performed using the compounds (II), (VII) and (VIII) in equimolar amounts or with slight excess amounts of (VII) and (VIII), in water as a solvent, in the presence of sodium hydrogensulfite, at room temperature or with heating, preferably with heating to reflux.

Other Processes:

Other compounds according to the invention can be formed by further conversion of substituent groups on above-described compounds.

The conversion of the substituents on $R^2$ or $R^3$ has not been mentioned in the foregoing detailed description but can be performed in the following conventional ways.

a) Compounds containing the cyano-lower alkyl group can be further converted, for example in accordance with the following representative schemes,to produce other compounds of the invention:

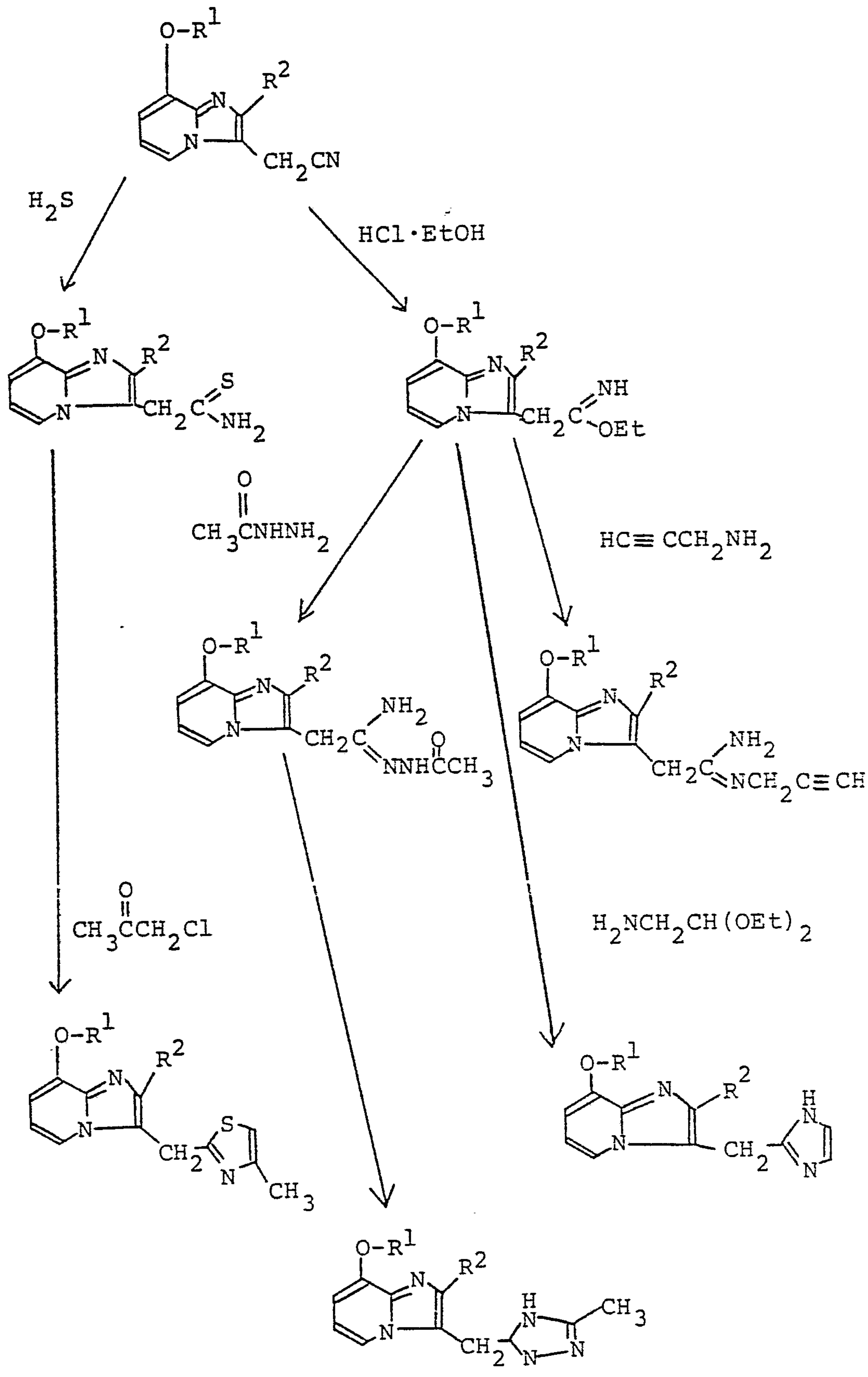


b) By N-alkylation of the compounds containing an amino group, other compounds containing a mono-or di-lower alkylamino group can be produced.

c) By reacting compounds containing a halogeno-lower alkyl group with thiourea and then reacting with alkalis and alkyl halides, compounds containing a lower alkyl group substituted with $-SR^8$ ($R^8$: a cyano-lower alkyl group or a lower alkynyl group) can be produced.

d) By reacting compounds containing a halogeno-lower alkyl group with metal lower alcoholates, compounds containing a lower alkyl group substituted with a lower alkoxy group can be produced.

e) By reacting compounds containing a hydroxy-lower alkyl group with lower acyl halides which may be substituted with a lower acyloxy group, compounds containing a lower acyloxyalkyl group which may be substituted with a lower acyloxy group can be produced.

f) By applying a Mannich reaction to compounds having a hydrogen atom on the imidazole ring, compounds containing a di-lower alkylaminomethyl group can be produced. These compounds can further be reacted with lower alkyl halides to produce quaternary ammonium salts of the compounds.

g) By reacting compounds containing an amino group with lower acyl halides or halogeno-sulfonic acid lower alkyl esters, compounds having a lower acylamino or lower alkylsulfonylamino group can be produced.

The compounds of the present invention produced by various processes as described above can be isolated and purified by conventional chemical operations such as extraction, crystallization, recrystallization, various column chromatography techniques, etc.

The compounds (I) and salts thereof provided by the present invention possess gastric acid secretion inhibitory activity, gastric mucous membrane protective activity (cytoprotective activity) and anti-ulcer activity and are useful as prophylactic and treating agents for gastric diseases,e.g. as gastric acid secretion inhibiting agents or prophylactic and treating agents for gastric ulcers.

The gastric acid secretion inhibitory activity and anti-ulcer activity of the compounds of the present invention have been confirmed by inhibitory activity ($IC_{50}$, 50% inhibition concentration) against $H^+,K^+$-adenosine triphosphatase [cf. Biochimica et Biophysica Acta, 728, 31-38 (1983)].

The gastric acid secretion inhibitory activity was also confirmed by the following experimental method.

Six male beagle dogs (13 to 17kg) with well-established Heidenhain pouches were used for the construction of gastric acid secretion response curves. Food was withheld but water allowed for 18 hours before each experiment. Histamine (60 µg/kg/hr) was administered subcutaneously 1 hour after drug administration. The pouch secretion was allowed to drain into a collection vessel which was changed every 15 minutes. Gastric juice volume was recorded, and acid concentration was determined by titration with 0.05N NaOH up to pH 7.0 using an automatic titrator (Hiranuma Sangyo Co., COMTITE-7).

The results obtained shown in the following table.

## Table

| Compound | $H^+,K^+$-ATPase Inhibitory Activity $IC_{50}$ (M) | Gastric Acid Secretion Inhibitory Rate (%) Pouch Dog 3 mg/kg p.o. |
|---|---|---|
| Known compound A 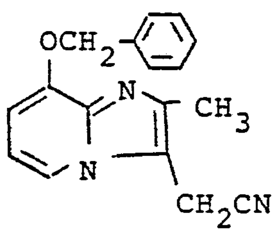 | 6.5 x $10^{-7}$ | 36 |
| Known compound B 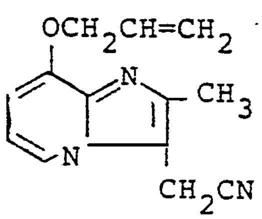 | 5.4 x $10^{-4}$ | 21 |
| Examples 16 and 38 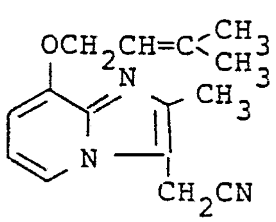 | 7.9 x $10^{-6}$ | 82 |

| Compound | $H^+, K^+$-ATPase Inhibitory Activity $IC_{50}$ (M) | Gastric Acid Secretion Inhibitory Rate (%) Pouch Dog 3 mg/kg p.o. |
|---|---|---|
| Example 11 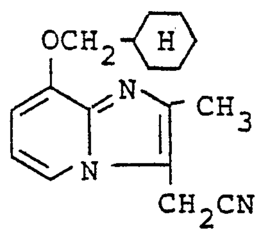 | $2.9 \times 10^{-6}$ | 67 |
| Example 12 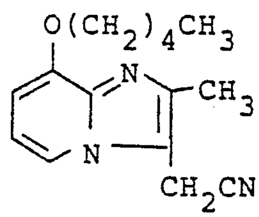 | $6.2 \times 10^{-6}$ | 61 |

Known compound A: compound of European Patent No. 33094
Known compound B: compound of European Patent No. 68378

The compounds (I) of the present invention and salts thereof can be used as they are or as pharmaceutical compositions obtained by mixing with conventional pharmaceutically acceptable carriers, excipients, etc. Administration may be oral (by tablets, capsules, powders, granules, pills or the like) or parenteral (by injections, syrups, ointments, suppositories, etc).

Dosage may be varied depending upon the patient concerned, route of administration and patient condition but generally totals 20 to 400 mg daily for an adult, which may be administered in 2 to 4 separate doses.

Hereafter the present invention is illustrated in more detail by the Examples but is not limited by these Examples.

At least some of the raw compounds used in the present invention are novel and included in the invention. Preparation of some such novel compounds is given in Reference Examples.

In the Examples, mp., Mass and $^1$HNMR indicate melting point, mass spectrum and $^1$H nuclear magnetic resonance spectrum, respectively.

An aqueous physiological saline solution of 1,000 mg/kg of a test compound, 3-cyanomethyl-2-methyl-8-[(3-methyl-2-butenyl)-oxy]imidazo[1,2-a]pyridine prepared by Example 16, was orally administered to one group of three male and two femal Fischer-rats (5-7 weeks) and they were observed for 24 hours but no instance of death was observed. That is, $LD_{50}$(p.o.) of the compound was higher than 1,000 mg/kg.

(Reference Example 1)

2-Amino-3-hexyloxypyridine:

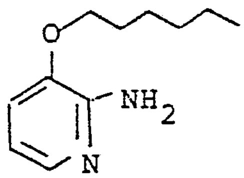

In a nitrogen gas flow, 2.0 g of sodium hydroxide was dissolved in 50 ml of methanol. Then 5.5 g of 2-amino-3-hydroxypridine was added to the solution under water chilling to dissolve it. Thereafter the solvent was evaporated off to dryness under reduced pressure. In a nitrogen gas flow, 70 ml of dry dimethylsulfoxide was added to the system and 9.08 g of hexyl bromide was added to the resulting suspension under water chilling followed by stirring at room temperature overnight. The solution was poured onto 400 ml of water. The mixture was extracted with ether (400 ml and 300 ml). After washing with water (300 ml), the extract was dried over anhydrous magnesium sulfate. The solvent was removed by distillation under reduced pressure and the obtained residue was washed with hexane to give 6.69 g of the product.
mp. 55 - 56°C; Mass (EI) m/z 194 ($M^+$)

The following compounds were obtained in a similar manner.

(Reference Example 2)

2-Amino-3-(2-butenyloxy)pyridine:

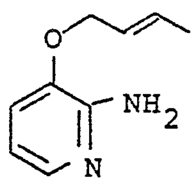

mp. 79 - 80°C/recrystallized from hexane Mass (EI) m/z 164 ($M^+$)

(Reference Example 3)

2-Amino-3-(3-methyl-2-butenyloxy)pyridine:

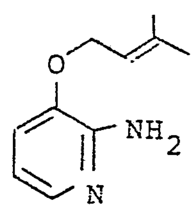

mp. 66 - 67°C/hexane Mass (EI) m/z 178 ($M^+$)

(Reference Example 4)

2-Amino-3-pentyloxypyridine:

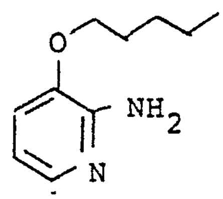

mp. 50 - 51 °C/hexane; Mass (EI) m/z 180 ($M^+$)

(Reference Example 5)

2-Amino-3-(cyclohexylmethoxy)pyridine:

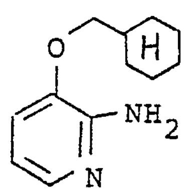

mp. 116 - 117°C/ether; Mass (EI) m/z 206 ($M^+$)

(Reference Example 6)

2-Amino-3-butoxypyridine:

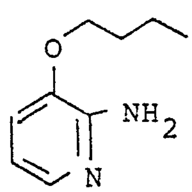

mp. 53 - 54°C/hexane; Mass (EI) m/z 166 ($M^+$)

(Reference Example 7)

2-Amino-3-octyloxypyridine:

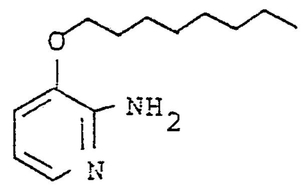

mp. 56 - 57°C/hexane; Mass (EI) m/z 222 ($M^+$)

(Reference Example 8)

2-Amino-3-(1-methylbutoxy)pyridine:

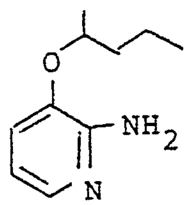

oily substance; Mass (EI) m/z 180 ($M^+$)
$^1$HNMR ($CDCl_3$, $\delta$)
0.8 - 1.1 (m,3H), 1.32 (d, 3H), 1.2 - 1.9 (m, 4H), 4.32 (dt, 1H), 6.54 (dd, 1H), 6.88 (dd, 1H), 7.62 (dd, 1H)

(Reference Example 9)

2-Amino-3-(2-ethylbutoxy)pyridine:

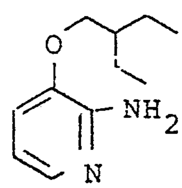

oily substance; Mass (EI) m/z 194 ($M^+$)
$^1$HNMR ($CDCl_3$, $\delta$)
0.92 (t, 6H), 1.1 - 1.8 (m, 5H), 3.86 (d, 2H), 6.56 (dd, 1H), 6.89 (dd, 1H), 7.62 (dd, 1H)

(Reference Example 10)

2-Amino-3-(ethoxy)pyridine:

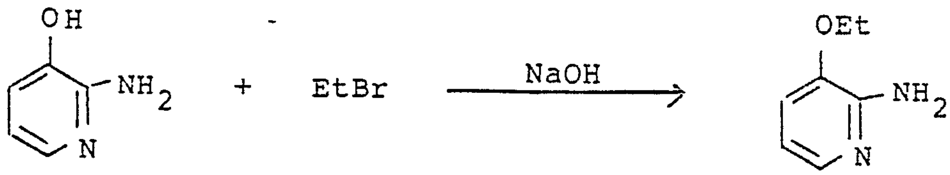


In 3 ml of water was dissolved 1.80 g of sodium hydroxide,and 50 ml of methanol and then 5.0 g of 2-amino-3-hydroxypyridine were added to the solution. After stirring at room temperature for 5 minutes, methanol was removed by distillation under reduced pressure. After evaporating to dryness, 100 ml of dimethylsulfoxide was added to the mixture followed by stirring at room temperature for 24 hours. The reaction mixture was poured onto 200 ml of water. The mixture was extracted with chloroform. The chloroform layer was washed, in sequence, with 5% sodium hydrogencarbonate aqueous solution and then water and dried over anhydrous magnesium sulfate. The solvent was distilled off. n-Hexane was added to the residue and the precipitated crystals were taken out by filtration to give 3.40 g of 2-amino-3-ethoxypyridine.

mp. 76 - 77°C; Mass (EI) m/z 138 ($M^+$)

The following compounds were synthesized in a similar manner.

(Reference Example 11)

2-Amino-3-propoxypyridine:

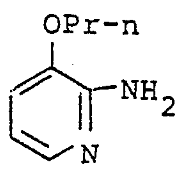

mp. 54 - 55°C; Mass (EI) m/z 152 ($M^+$)

(Reference Example 12)

2-Amino-3-(2-methylpropoxy)pyridine:

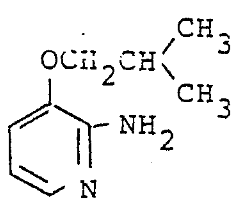

mp. 45 - 46°C/hexane; Mass (EI) m/z 166 ($M^+$)

(Reference Example 13)

2-Amino-3-(2-cyclohexylethoxy)pyridine:

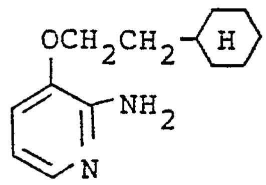

mp. 99 - 100°C Mass (EI) m/z 220 ($M^+$)

Example 1

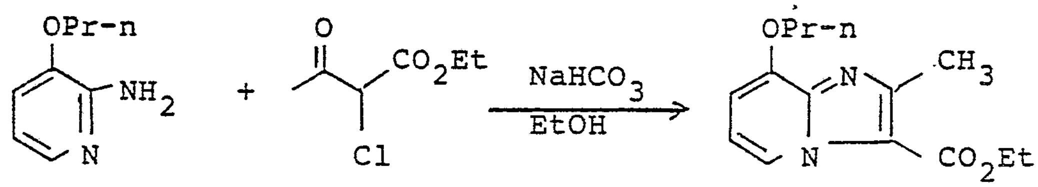


In 200 ml of ethanol was dissolved 24.0 g of ethyl 2-chloroacetoacetate and, 11.1 g of 2-amino-3-propoxypyridine was added to the solution. After adding 6.2 g of sodium hydrogencarbonate, the mixture was heated to reflux for 16 hours and the solvent was then distilled off. 100 ml of water was added to the residue and the resultant mixture extracted with chloroform. The extract was dried over anhydrous magnesium sulfate, and the solvent was distilled off. The residue was purified by silica gel column chromatography using hexane:ethyl acetate (1:1) to give 4.0 g of 3-ethoxycarbonyl-2-methyl-8-propoxyimidazo [1,2-a]pyridine.
mp. 56-57 °C; Mass (EI) m/z 262 ($M^+$)

Example 2

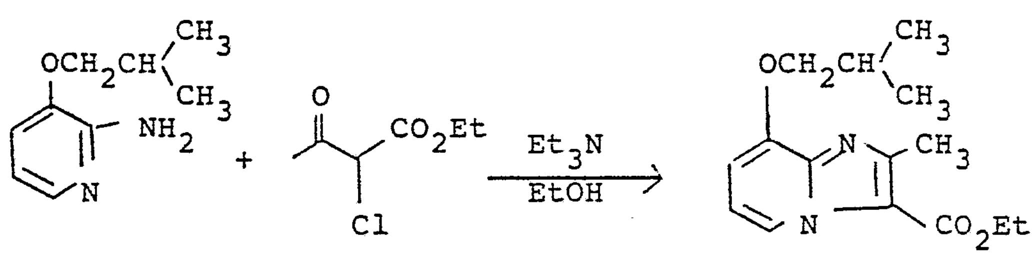


In 30 ml of ethanol was dissolved 1.0 g of ethyl 2-chloroacetoacetate and, 0.8 g of 2-amino-3-(2-methylpropoxy)pyridine and then 0.50 g of triethylamine were added to the solution. After heating to reflux for 6 hours, 1.0 g of ethyl 2-chloroacetoacetate was added to the mixture followed by heating to reflux for 16 hours. After cooling, ethanol was distilled off under reduced pressure. The residue was dissolved in chloroform. The solution was washed with water and dried over anhydrous magnesium sulfate. The solvent was distilled off. The residue was purified by silica gel column chromatography using hexane : ethyl acetate (4 : 1) to give 0.45 g of 3-ethoxycarbonyl-2-methyl-8-(2-methylpropoxy)imidazo[1,2-a]-pyridine.
mp. 59 - 60°C; Mass (EI) m/z 276 ($M^+$) Elemental analysis (as $C_{15}H_{20}N_2O_3$)

| | C | H | N |
|---|---|---|---|
| Calcd. (%) | 65.20 | 7.30 | 10.14 |
| Found (%) | 64.98 | 7.38 | 10.12 |

Example 3

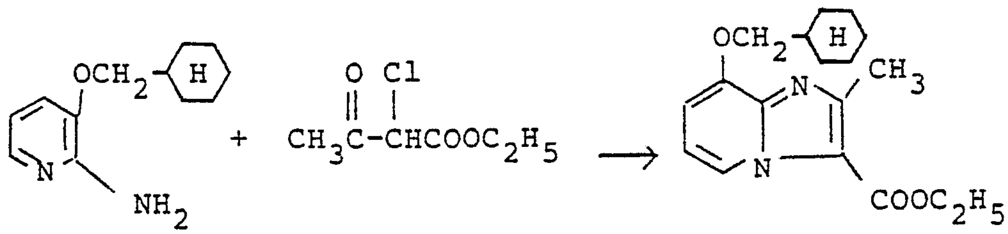

In 100 ml of toluene was dissolved 5.76 g of 2-amino-3-cyclohexylmethoxypyridine, 5.0 g of ethyl 2-chloroacetoacetate and 3.09 g of triethylamine. The solution was heated to reflux for 24 hours. To the reaction solution was added 100 ml of ether. The mixture was washed with water. The organic phase was dried over anhydrous magnesium sulfate and the solvent was distilled off. Recrystallization from ether-n-hexane gave 5.67 g of 8-cyclohexylmethoxy-3-ethoxycarbonyl-2-methylimidazo[1,2-a]pyridine of mp. 108 - 109°C.

Mass (FAB) m/z 317 ($M^+$ + 1)
Elemental analysis (as $C_{18}H_{24}N_2O_3$)

| | C | H | N |
|---|---|---|---|
| Calcd. (%) | 68.33 | 7.65 | 8.85 |
| Found (%) | 68.21 | 7.67 | 8.79 |

The following compound was obtained in a similar manner to Example 3.

Example 4

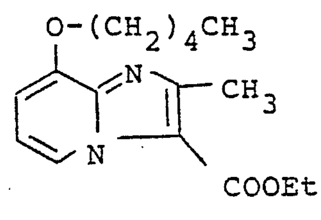

3-Ethoxycarbonyl-2-methyl-8-pentyloxyimidazo-[1,2-a]pyridine
mp. 59 - 60 °C; Mass (FAB) m/z 291 ($M^+$ + 1)

Example 5

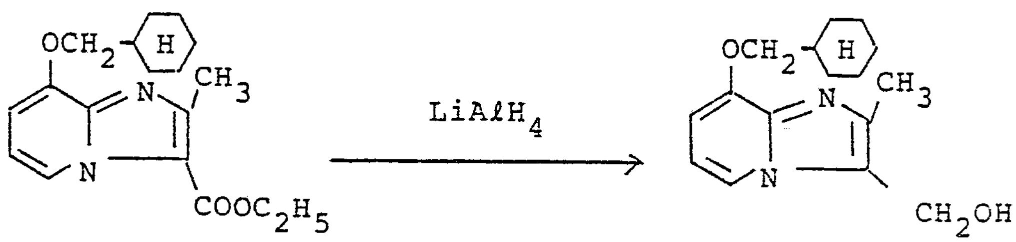


In an argon gas flow, 30 ml of anhydrous tetrahydrofuran was added to 0.78 g of lithium aluminum hydride. The mixture was stirred and cooled with ice water at 0 to 10°C,and a solution of 5.48 g of 8-cyclohexylmethoxy-3-ethoxycarbonyl-2-methylimidazo[1,2-a]pyridine in 50 ml of anhydrous tetrahydrofuran was dropwise added thereto. After completion of the dropwise addition, the mixture was stirred at 0 to 10°C for an hour. By carefully adding 7 ml of water, excess lithium aluminum hydride was decomposed and 100 ml of ether was added to revert to room temperature, where insoluble matters were removed by filtration. After washing with saturated sodium chloride aqueous solution, the filtrate was dried over anhydrous magnesium sulfate. The solvent was distilled off and the residue was recrystallized from ether-n-hexane to give 4.22 g of 8-cyclohexylmethoxy-3-hydroxymethyl-2-methylimidazo[1,2-a]pyridine (mp. 146°C).

Mass (FAB) m/z 275 ($M^+ + 1$)

Elemental analysis (as $C_{16}H_{22}N_2O_2$)

| | C | H | N |
|---|---|---|---|
| Calcd. (%) | 70.04 | 8.08 | 10.21 |
| Found (%) | 70.06 | 8.09 | 10.18 |

The following compounds were obtained in a similar manner to Example 5.

Example 6

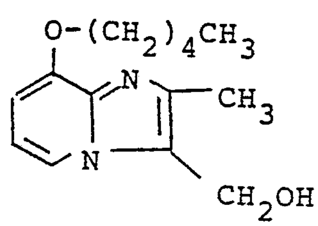

3-Hydroxymethyl-2-methyl-8-pentyloxyimidazo-[1,2-a]pyridine

mp. 116 - 117°C; Mass (FAB) m/z 249 ($M^+ + 1$)

Elemental analysis (as $C_{14}H_{20}N_2O_2$)

| | C | H | N |
|---|---|---|---|
| Calcd. (%) | 67.72 | 8.12 | 11.28 |
| Found (%) | 67.62 | 8.09 | 11.19 |

Example 7

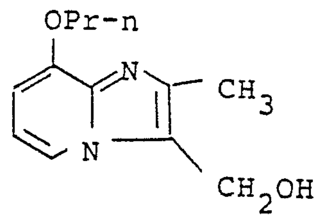

3-Hydroxymethyl-2-methyl-8-propoxyimidazo-[1,2-a]pyridine
mp. 136 - 137°C; Mass (EI) m/z 220 ($M^+$)
Elemental analysis (as $C_{12}H_{16}N_2O_2$)

| | C | H | N |
|---|---|---|---|
| Calcd. (%) | 65.43 | 7.32 | 12.72 |
| Found (%) | 65.26 | 7.28 | 12.68 |

Example 8

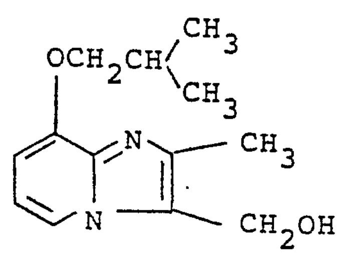

3-Hydroxymethyl-2-methyl-8-(2-methylpropoxy)-imidazo[1,2-a]pyridine
mp. 130 - 132°C; Mass (EI) m/z 234 ($M^+$)
Elemental analysis (as $C_{13}H_{18}N_2O_2$)

| | C | H | N |
|---|---|---|---|
| Calcd. (%) | 66.64 | 7.74 | 11.96 |
| Found (%) | 66.46 | 7.72 | 11.79 |

Example 9

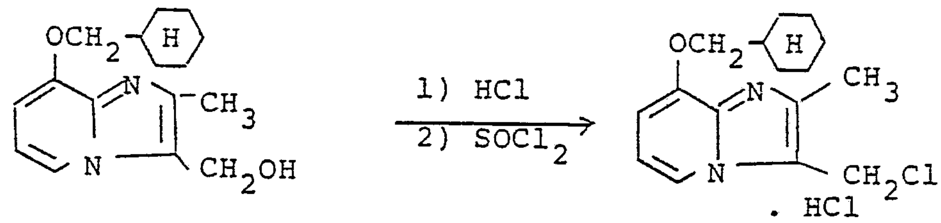


In ethanol was treated 3.3 g of 8-cyclohexylmethoxy-3-hydroxy-methyl-2-methylimidazo[1,2-a]pyridine was treated with HCℓ to form the hydrochloride.

Thereafter the hydrochloride was dissolved in 30 ml of thionyl chloride and the solution was stirred at room temperature for 3 hours.

An excess of thionyl chloride was distilled off under reduced pressure and the residue was washed with ethanol-ether to give 3.69 g of 3-chloromethyl-8-cyclohexylmethoxy-2-methylimidazo[1,2-a]pyridine hydrochloride of mp. 144 - 146°C.

The following compound was obtained in a similar manner.

Example 10

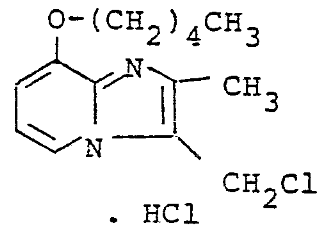

3-Chloromethyl-2-methyl-8-pentyloxyimidazo-[1,2-a]pyridine hydrochloride: mp. 96 - 98°C

Example 11

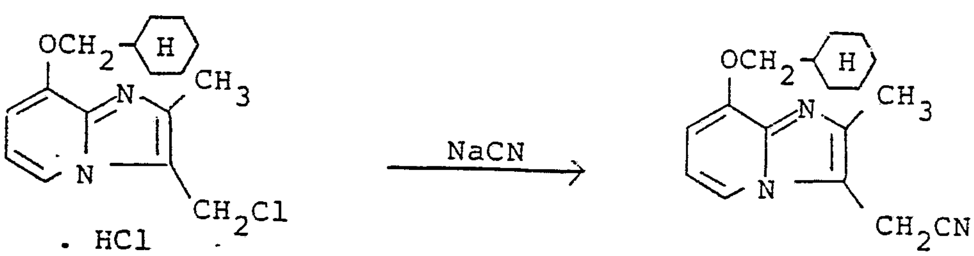


To 20 ml of anhydrous dimethylsulfoxide was added 2.19 g of sodium cyanide. Under ice cooling, a solution of 3.69 g of 3-chloromethyl-8-cyclohexylmethoxy-2-methylimidazo[1,2-a]pyridine hydrochloride in 30 ml of anhydrous dimethylsulfoxide was dropwise added to the mixture. The temperature was reverted to room temperature followed by stirring for 3 hours.

The reaction solution was added to 300 ml of 3% sodium bicarbonate aqueous solution. The mixture was extracted with ethyl acetate (150 ml x 3). After washing with water, the organic phase was dried over anhydrous magnesium sulfate. The solvent was distilled off under reduced pressure and the residue was purified by silica gel column chromatography using a solvent mixture of ethyl acetate-n-hexane. Recrystallization from ether-n-hexane gave 1.3 g of 3-cyanomethyl-8-cyclohexylmethoxy-2-methylimidazo-[1,2-a]-pyridine of mp. 145 to 146°C.
Mass (EI) m/z 283 ($M^+$)
Elemental analysis (as $C_{17}H_{21}N_3O$)

| | C | H | N |
|---|---|---|---|
| Calcd. (%) | 72.06 | 7.47 | 14.83 |
| Found (%) | 72.09 | 7.47 | 14.71 |

The following compound was obtained in a similar manner.

Example 12

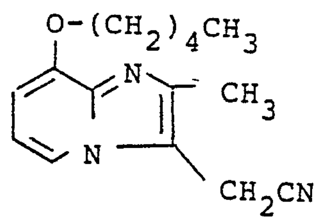

3-Cyanomethyl-2-methyl-8-pentyloxyimidazo-[1,2-a]pyridine
mp. 124 - 125°C; Mass (EI) m/z 257 ($M^+$)
Elemental analysis (as $C_{15}H_{19}N_3O$)

| | C | H | N |
|---|---|---|---|
| Calcd. (%) | 70.01 | 7.44 | 16.33 |
| Found (%) | 69.79 | 7.46 | 16.05 |

Example 13

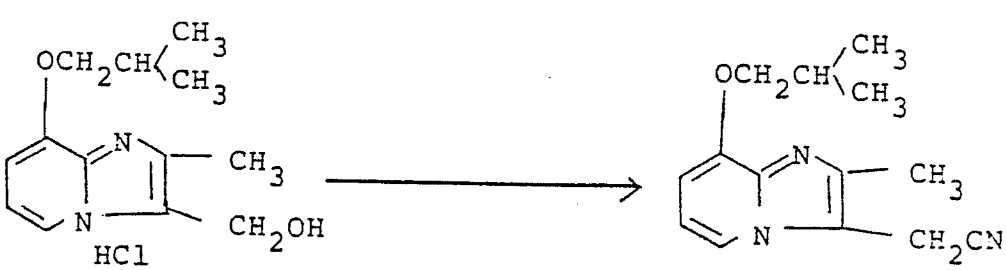

To 5 ml of thionyl chloride was added 1.78 g of 3-hydroxymethyl-2-methyl-8-(2-methylpropoxy)-imidazo[1,2-a]pyridine hydrochloride. The mixture was stirred at room temperature for 5 hours. Thionyl chloride was distilled off under reduced pressure. Ether was added to the residue to cause crystallization. In 30 ml of dimethylsulfoxide was dissolved 1.20 g of sodium cyanide, to which a solution of the crude crystals obtained above in dimethylsulfoxide was dropwise added under ice cooling in an argon gas flow. The mixture was stirred at room temperature for 3 hours. The reaction mixture was added to 300 ml of 3%

sodium hydrogencarbonate aqueous solution and extracted with ethyl acetate. The extract was washed with water and dried over anhydrous magnesium sulfate. The solvent was distilled off. The residue was purified by silica gel column chromatography using ethyl acetate as a developing solvent. Recrystallization from hexane-ethyl acetate gave 0.50 g of 3-cyanomethyl-2-methyl-8-(2-methyl propoxy)imidazo[1,2-a]pyridine.

mp. 113 - 114°C; Mass (EI) m/z 243 ($M^+$)

Elemental analysis (as $C_{14}H_{17}N_3O$)

| | C | H | N |
|---|---|---|---|
| Calcd. (%) | 69.11 | 7.04 | 17.27 |
| Found (%) | 69.00 | 7.08 | 17.07 |

Example 14

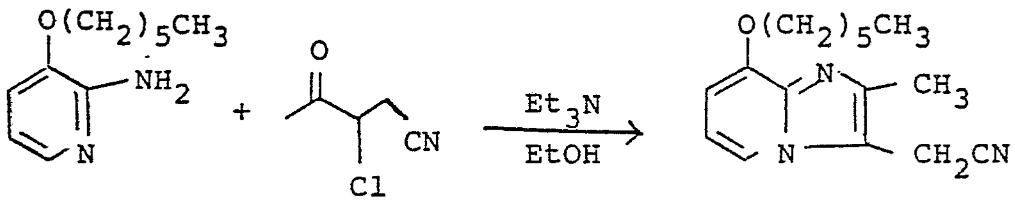


In a nitrogen gas flow, 1.32 g of 3-chloro-4-oxopentanenitrile and 1.55 g of 2-amino-3-hexyloxy-pyridine and then 0.81 g of triethylamine were added to 40 ml of ethanol. After stirring under reflux overnight, the solvent was distilled off under reduced pressure. A sodium carbonate aqueous solution was added to the residue and the solution was extracted with methylene chloride. After drying over anhydrous magnesium sulfate, the solvent was distilled off under reduced pressure. The residue was purified by column chromatography and crystallized from ether to give 1.30 g of 3-cyanomethyl-8-n-hexyloxy-2-methyl-imidazo[1,2-a]pyridine.

mp. 119 - 120 °C; Mass (EI) m/z 271 ($M^+$)

Elemental analysis (as $C_{16}H_{21}N_3O$)

| | C | H | N |
|---|---|---|---|
| Calcd. (%) | 70.82 | 7.80 | 15.48 |
| Found (%) | 70.59 | 7.75 | 15.38 |

The following compounds were obtained in a similar manner.

Example 15

8-((E)-2-Butenyloxy)-3-cyanomethyl-2-methylimidazo-[1,2-a]pyridine:

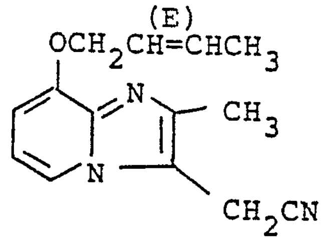

mp. 93 - 95°C/ethyl acetate-ether
Mass (EI) m/z 241 ($M^+$)
Elemental analysis (as $C_{14}H_{15}N_3O$)

| | C | H | N |
|---|---|---|---|
| Calcd. (%) | 69.69 | 6.27 | 17.41 |
| Found (%) | 69.54 | 6.30 | 17.36 |

Example 16

3-Cyanomethyl-2-methyl-8-[(3-methyl-2-butenyl)-oxy]imidazo[1,2-a]pyridine:

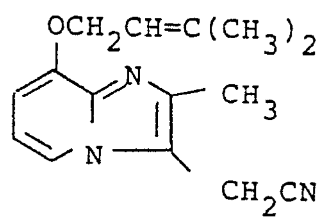

mp. 118 - 119°C/ether
Mass (EI) m/z 255 ($M^+$)
Elemental analysis (as $C_{15}H_{17}N_3O$)

| | C | H | N |
|---|---|---|---|
| Calcd. (%) | 70.56 | 6.71 | 16.46 |
| Found (%) | 70.45 | 6.77 | 16.41 |

Example 17

3-Cyanomethyl-2-methyl-8-(1-methylbutoxy)imidazo[1,2-a]pyridine 2/3 fumarate:

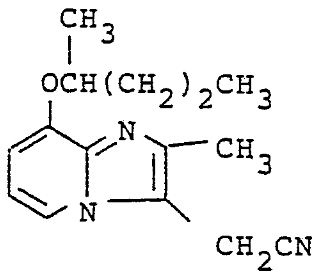

.2/3 fumarate

mp. 135 - 140°C/ethanol-ether
Mass (EI) m/z 257 ($M^+$)
Elemental analysis (as $C_{15}H_{19}N_3O.2/3\ (C_4H_4O_4)$)

| | C | H | N |
|---|---|---|---|
| Calcd. (%) | 63.40 | 6.52 | 12.55 |
| Found (%) | 63.46 | 6.58 | 12.73 |

Example 18

3-Cyanomethyl-8-[(2-ethylbutoxy)]imidazo[1,2-a]pyridine:

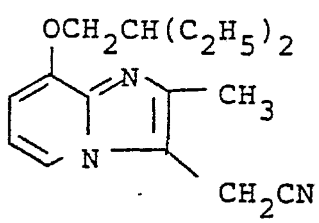

mp. 123 - 124°C/ether
Mass (EI) m/z 271 ($M^+$)
Elemental analysis (as $C_{16}H_{21}N_3O$)

| | C | H | N |
|---|---|---|---|
| Calcd. (%) | 70.82 | 7.80 | 15.48 |
| Found (%) | 70.64 | 7.78 | 15.42 |

Example 19

8-Butoxy-3-cyanomethyl-2-methylimidazo[1,2-a]pyridine:

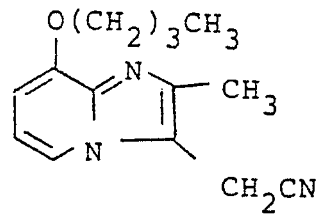

mp. 105 - 107°C/ether
Mass (EI) m/z 243 ($M^+$)
Elemental analysis (as $C_{14}H_{17}N_3O$)

| | C | H | N |
|---|---|---|---|
| Calcd. (%) | 69.11 | 7.04 | 17.27 |
| Found (%) | 69.13 | 7.13 | 17.23 |

Example 20

3-Cyanomethyl-2-methyl-8-octyloxyimidazo[1,2-a]pyridine:

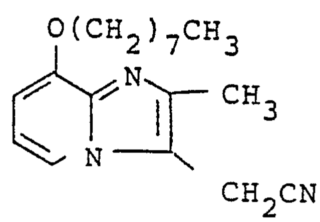

mp. 90.5 - 91.5°C/ether-hexane
Mass (EI) m/z 299 ($M^+$)
Elemental analysis (as $C_{18}H_{25}N_3O$)

| | C | H | N |
|---|---|---|---|
| Calcd. (%) | 72.21 | 8.42 | 14.03 |
| Found (%) | 72.18 | 8.40 | 14.01 |

Example 21

3-Cyanomethyl-8-(2-cyclohexylethoxy)-2-methylimidazo[1,2-a]pyridine:

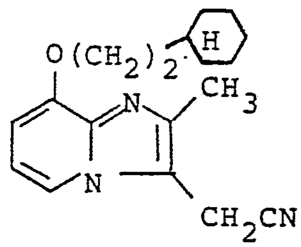

mp. 142 - 144°C; Mass (EI) m/z 297 ($M^+$)
Elemental analysis (as $C_{18}H_{23}N_3O$)

| | C | H | N |
|---|---|---|---|
| Calcd. (%) | 72.70 | 7.80 | 14.13 |
| Found (%) | 72.65 | 7.75 | 14.06 |

Example 22

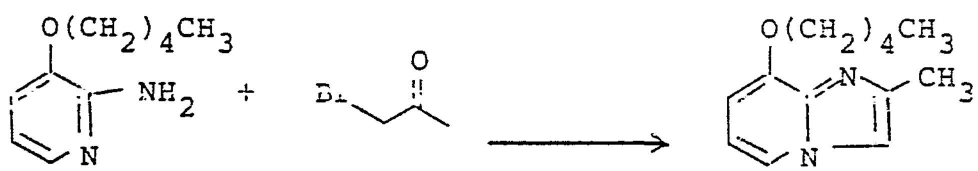

To 30 ml of ethanol were added 3.60 g of 2-amino-3-pentyloxypyridine and 2.0 ml of bromoacetone. After refluxing for 2 hours, 1.0 ml of bromoacetone was added to the mixture under water chilling followed by stirring to reflux overnight. The solvent was distilled off under reduced pressure. A sodium carbonate aqueous solution was added to the residue followed by extraction with chloroform. After drying over anhydrous magnesium sulfate, the solvent was distilled off under reduced pressure. The residue was purified by column chromatography and then recrystallized from hexane to give 2.04 g of 2-methyl-8-pentyloxyimidazo[1,2-a]pyridine.
mp. 68 - 69°C; Mass (EI) m/z 218 ($M^+$)

Example 23

8-(cyclohexylmethyloxy)-2-methylimidazo[1,2-a]pyridine was synthesized in a manner similar to Example 22.

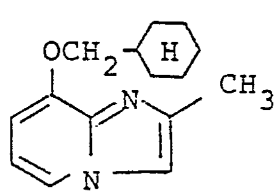

oily substance: Mass (EI) m/z 244 ($M^+$)

$^1$HNMR ($CDCl_3$, δ)
0.8 -2.1 (m, 11H), 2.48 (s, 3H), 3.89 (d, 2H), 6.36 (dd, 1H), 6.60 (dd, 1H), 7.28 (s, 1H), 7.64 (dd, 1H)

Example 24

8-(Cyclohexylmethyloxy)-2-methyl-3-nitrosoimidazo[1,2-a]pyridine:

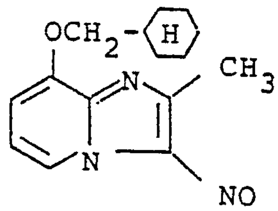

To 28 ml of dioxane was added 2.8 g of 8-(cyclohexylmethyloxy)-2-methylimidazo[1,2-a]pyridine. After 20.75 g of isoamyl nitrite was added thereto at 50°C over 5 minutes, the mixture was stirred to reflux for 25 minutes. After concentration, the concentrate was purified by column chromatography and crystallized from hexane to give 1.27 g of the desired product.
mp. 88 - 89°C; Mass (EI) m/z 273 ($M^+$)

Example 25

2-Methyl-3-nitroso-8-pentyloxyimidazo[1,2-a]pyridine:

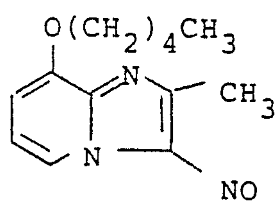

The compound was synthesized in a manner similar to Example 24.
mp. 67 - 68°C/hexane; Mass (EI) m/z 247 ($M^+$)

Example 26

3-Amino-8-(cyclohexylmethyloxy)-2-methylimidazo[1,2-a]pyridine fumarate:

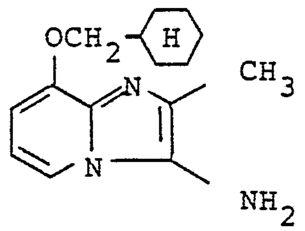

. fumarate

In a mixture of 14.5 ml of acetic acid and 11.5 ml of water was dissolved 1.27 g of 8-(cyclohexylmethyloxy)-2-methyl-3-nitrosoimidazo[1,2-a]pyridine. After 1.50 g of zinc powder was added to the solution over approximately 30 minutes, stirring was continued for 30 minutes. Insoluble matters were filtered off through Celite and the filtrate was concentrated to dryness. A sodium carbonate aqueous solution was added to the residue followed by extraction with methylene chloride. After drying, the solvent was concentrated and distilled off. The residue was dissolved in a small quantity of ethanol and an ethanolic solution of 0.45 g of fumaric acid was added to the solution. The formed salt was taken by filtration and then dried to give 0.85 g of the desired product.
mp. 188 - 190°C.
Elemental analysis (as $C_{15}H_{21}N_3O.C_4H_4O_4$)

| | C | H | N |
|---|---|---|---|
| Calcd. (%) | 60.79 | 6.71 | 11.19 |
| Found (%) | 60.53 | 6.69 | 11.00 |

Example 27

3-Amino-2-methyl-8-pentyloxyimidazo[1,2-a]pyridine fumarate:

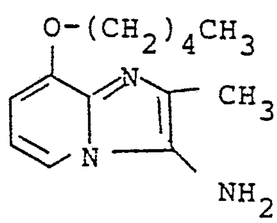

. fumarate

This compound was synthesized in a manner similar to example 26.
mp. 183 - 185°C/methanol-ethanol
Mass (EI) m/z 233 ($M^+$)
Elemental analysis (as $C_{13}H_{19}N_3O.C_4H_4O_4$)

| | C | H | N |
|---|---|---|---|
| Calcd. (%) | 58.44 | 6.64 | 12.03 |
| Found (%) | 58.35 | 6.61 | 11.97 |

Example 28

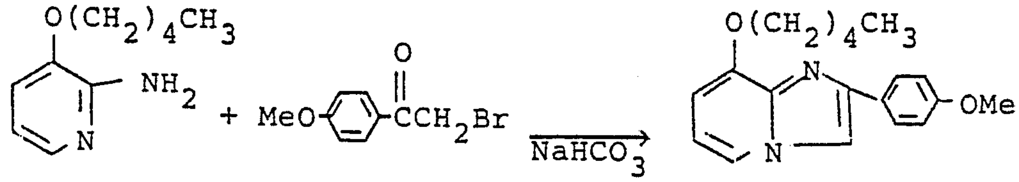


In 30 ml of ethanol were dissolved 1.8 g of 2-amino-3-pentyloxypyridine, 2.29 g of 4-methoxyphenacyl bromide and 0.84 g of sodium bicarbonate. The mixture was heated to reflux for 2 hours.

The solvent was distilled off under reduced pressure. After washing the obtained crystals with water, recrystallization was performed for ethyl acetate to give 0.8 g of 2-(p-methoxyphenyl)-8-pentyloxyimidazo-[1,2-a]pyridine with a melting point of 118 to 119°C.

Mass (EI) m/z 310 ($M^+$)

Elemental analysis (as $C_{19}H_{22}N_2O_2$)

| | C | H | N |
|---|---|---|---|
| Calcd. (%) | 73.52 | 7.14 | 9.03 |
| Found (%) | 73.65 | 7.11 | 9.11 |

Example 29

The following compound was obtained in a manner similar to Example 28 except for using 2-bromo-3-butanone

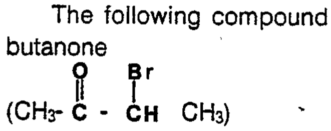

in place of 4-methoxyphenacyl bromide.

2,3-Dimethyl-8-pentyloxyimidazo[1,2-a]pyridine:

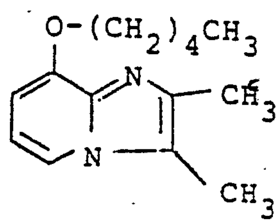

mp. 53 - 55°C

Mass (EI) m.z 232 ($M^+$), 175, 162.

Example 30

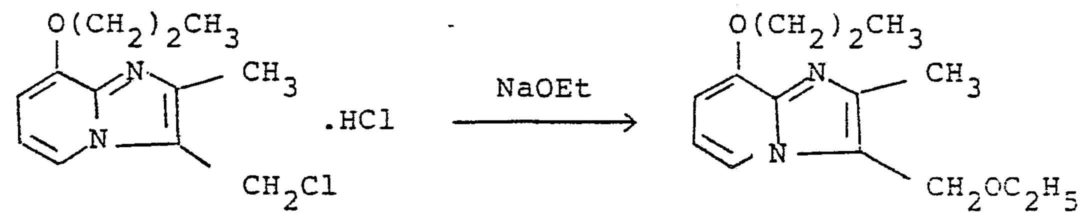


In a solution of 250 mg of metallic sodium in 30 ml of ethanol was dissolved 1.0 g of 8-propoxy-3-chloromethyl-2-methylimidazo[1,2-a]pyridine hydro chloride. The mixture was stirred at room temperature for 24 hours. The solvent was distilled off under reduced pressure. The residue was purified by silica gel column chromatography using an ethyl actate-n-hexane solvent mixture as a developing solvent. Treatment of the resulting compound with fumaric acid gave 0.2 g of 8-propoxy-3-ethoxymethyl-2-methylimidazo[1,2-a]pyridine 1/2 fumarate with a melting point 160 to 161°C.
Mass (EI) m/z 248 ($M^+$)
Elemental analysis (as $C_{14}H_{20}N_2O_2.1/2\ C_4H_4O_4$)

| | C | H | N |
|---|---|---|---|
| Calcd. (%) | 62.73 | 7.24 | 9.14 |
| Found (%) | 62.60 | 7.08 | 9.33 |

Example 31

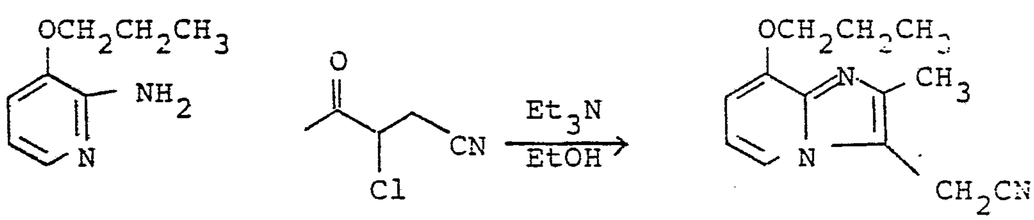


2-Amino-3-n-propyloxypyridine, 1.50 g (9.8 mmols) and 2.0 g (15.2 mmols) of 3-chloro-4-oxopentanenitrile were heated to reflux in 40 ml of ethanol for 16 hours in the presence of 1.0 g (9.8 mmols) of triethylamine. Then, ethanol was distilled off and the residue was separated by silica gel column chromatography ($CHCl_3$) and recrystallized from hexane-ethyl acetate to give 0.30 g (13.3%) of 3-cyanomethyl-2-methyl-8-n-propyloxyimidazo[1,2-a]pyridine.
mp. 106 - 107°C; Mass (EI) m/z ($M^+$)
Elemental analysis (as $C_{13}H_{15}N_3O$)

| | C | H | N |
|---|---|---|---|
| Calcd. (%) | 68.10 | 6.59 | 18.33 |
| Found (%) | 67.92 | 6.59 | 18.21 |

Example 32

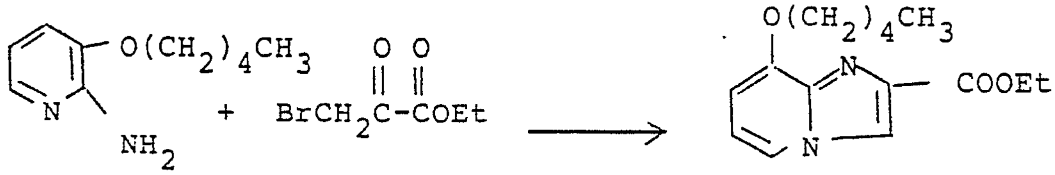

2-Amino-3-n-pentyloxpyridine 10 g (55.48 mmols), 12.98 g (66.56 mmols) of ethyl bromopyruvate and 7.0 g (83.33 mmols) of sodium bicarbonate were stirred in 200 ml of ethanol at room temperature for 2 hours and heated to reflux for a further 2 hours.After the solvent was distilled off under reduced pressure, the residue was dissolved in ether and the solution was washed with water. After drying over anhydrous magnesium sulfate, the ethereal phase was concentrated to dryness under reduced pressure to give 8.0 g (yield, 52.2%) of 8-n-pentyloxy-2-ethoxycarbonylimidazo[1,2-a]pyridine crystals with a melting point of 120 to 121°C.

Mass (EI) m/z 276 ($M^+$), 231, 219, 206.

The following compound was obtained in a similar manner.

Example 33

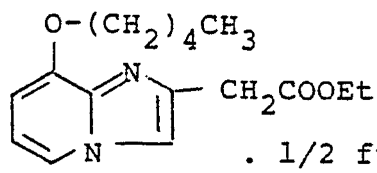

. 1/2 fumarate

Ethyl (8-n-pentyloxyimidazo[1,2-a]pyridin-2-yl) acetate 1/2 fumarate

mp. 78°C

Elemental analysis (as $C_{16}H_{22}N_2O_3.1/2\ C_4H_4O_4$)

| | C | H | N |
|---|---|---|---|
| Calcd. (%) | 62.05 | 6.94 | 8.04 |
| Found (%) | 61.97 | 6.88 | 8.03 |

Example 34

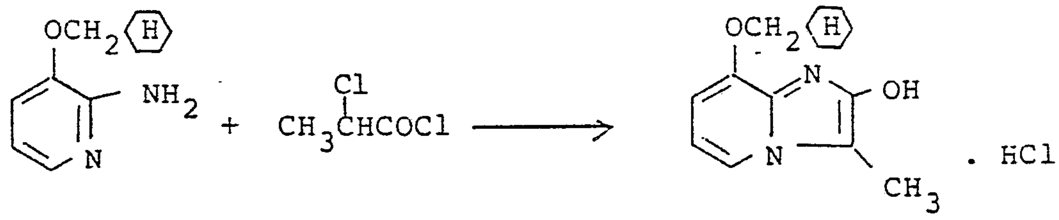

In 5 ml of dry ether was dissolved 0.72 g of 2-amino-3-cyclohexylmethoxypyridine. After 0.28 g of pyridine was added to the solution, a solution of 0.34 ml of 2-chloropropanoyl chloride in 2 ml of ether was added to the mixture at -10°C. After stirring at -10°C for an hour, the system was washed with chilled water and dried. Then the solvent was distilled off under reduced pressure. The residue was recrystallized from ether-hexane. The thus obtained 2-chloro-(N-cyclohexylmethoxy-2-pyridyl)propanamide hydrochloride was reacted with heating at 120°C for 2 hours. The residue was recrystallized from ethanol-ether to give 0.35 g of the desired product, 8-cyclohexylmethoxy-2-hydroxy-3-methylimidazo[1,2-a]pyridine hydrochloride.
mp. 167 - 169°C; Mass (EI) m/z 260 ($M^+$)
Elemental analysis (as $C_{15}H_{20}N_2O_2$. HCl•1/4 $H_2O$)

| | C | H | N | Cl |
|---|---|---|---|---|
| Calcd. (%) | 59.79 | 7.19 | 9.30 | 11.75 |
| Found (%) | 59.75 | 7.12 | 9.22 | 11.65 |

Example 35

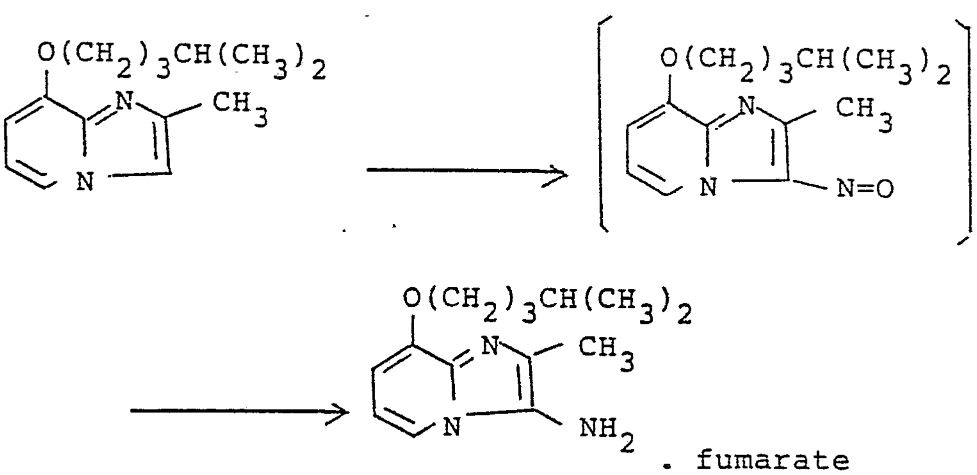

To 150 ml of dioxane was added 8.0 g (34.4 mmols) of 8-iso-hexyloxy-2-methylimidazo[1,2-a]pyridine and, 68.5 ml (0.51 mols) of isoamyl nitrite was added thereto. After heating to reflux for 30 minutes, the solvent was distilled off and 200 ml of acetic acid and 100 ml of water were added to the residue. To the mixture was added 9.0 g (0.137 mols) of Zn at room temperature. After stirring for 4 hours, the mixture was filtered and the filtrate was distilled off. Chloroform and water were added to the residue. The mixture was made alkaline by a sodium hydroxide aqueous solution and filtered. The filtrate was extracted with

chloroform and dried over anhydrous magnesium sulfate. The solvent was distilled off and the residue was purified by silica gel column chromatography (ethyl acetate), to which 0.75 g (6.5 mmols) of fumaric acid was added to convert into the fumarate. Recrystallization from methanol gave 1.20 g (9.6%) of 3-amino-8-iso-hexyloxy-2-methylimidazo[1,2-a]pyridine fumarate.
mp. 194 to 196°C; Mass (EI) m/z 247 ($M^+$)
Elemental analysis (as $C_{14}H_{21}N_3O.C_4H_4O_4$)

| | C | H | N |
|---|---|---|---|
| Calcd. (%) | 59.49 | 6.93 | 11.56 |
| Found (%) | 59.51 | 6.91 | 11.58 |

Example 36

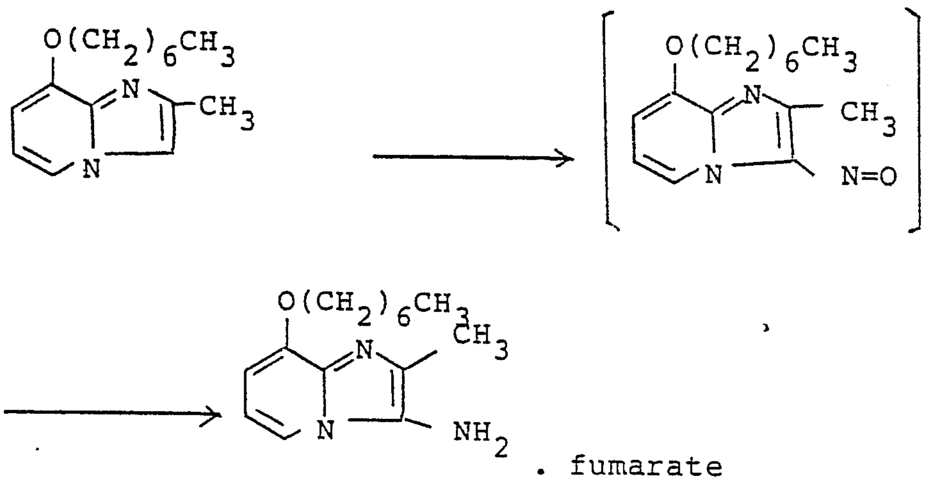

· fumarate

In 60 ml of dioxane was dissolved 1.90 g (7.71 mmols) of 8-n-heptyloxy-2-methylimidazo[1,2-a]pyridine and 20.7 ml (0.154 mols) of isoamyl nitrite was added to the solution. After heating to reflux for 30 minutes, the solvent was distilled off under reduced pressure and 30 ml of acetic acid and 10 ml of water were added to the residue. To the mixture was added 2.1 g (32.1 mmols of Zn. After stirring for 2 hours at room temperature, the solvent was distilled off. To residue were added 50 ml of methylene chloride and 20 ml water. The system was made alkaline with NaOH aqueous solution. After filtering through Celite, washing with methanol and concentrating to some degree, the concentrate was extracted with methylene chloride and dried over anhydrous magnesium sulfate. After the solvent was distilled off, the residue was purified by silica gel column chromatography. The purified compound was converted into the fumarate in conventional manner and recrystallized from ethanol to give 0.40 g (20%) of 3-amino-8-n-heptyloxy-2-methylimidazo-[1,2-a]pyridine fumarate.
mp. 171 - 173°C; Mass (EI) m/z 261 ($M^+$)
Elemental analysis (as $C_{15}H_{23}N_3O.C_4H_4O_4$)

| | C | H | N |
|---|---|---|---|
| Calcd. (%) | 60.46 | 7.21 | 11.13 |
| Found (%) | 60.49 | 7.26 | 11.12 |

Example 37

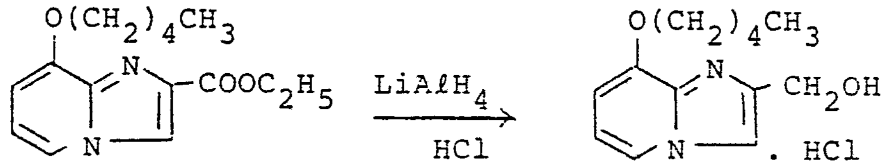


In an argon gas flow, 1.5 g (39.5 mmols) of lithium aluminum hydride was suspended in 100 ml of dry tetrahydrofuran and a solution of 16 g (57.9 mmols) of 8-n-pentyloxy-2-ethoxycarbonylimidazo-[1,2-a]-pyridine in 300 ml of dry tetrahydrofuran was dropwise added to the suspension. After stirring at room temperature for 30 minutes, the mixture was heated at 65°C for 20 minutes. Approximately 15 ml of water was dropwise added to the system carefully. Insoluble matter were filtered off. After the solvent was distilled off under reduced pressure, the residue was treated with hydrochloric acid in ethanol and recrystallized from acetone to give 13.9 g (yield, 88.7%) of 8-n-pentyloxy-2-hydroxymethylimidazo[1,2-a]pyridine hydrochloride with a melting point of 102 to 103°C.

Elemental analysis (as $C_{13}H_8N_2O_2$ HCl)

| | C | H | N | Cl |
|---|---|---|---|---|
| Calcd. (%) | 57.67 | 7.07 | 10.35 | 13.09 |
| Found (%) | 57.67 | 7.06 | 10.36 | 13.33 |

Example 38

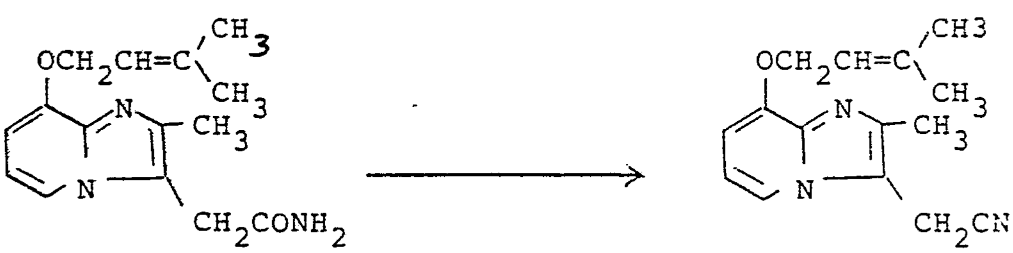

a) To 2.5 ℓ of methylene chloride were added 248 g of 3-carbamoyl-2-methyl-8-[(3-methyl-2-butenyl)-oxy]imidazo[1,2-a]pyridine and 204 ml of pyridine. Then 297 ml of trifluoroacetic anhydride was dropwise added to the mixture at 0°C. After completion of the reaction, the reaction mixture was poured into 5 ℓ of cold saturated sodium hydrogencarbonate aqueous solution followed by fractionation. After drying over anhydrous magnesium sulfate, the organic phase was concentrated to dryness under reduced pressure. The residue was recrystallized from methylene chlorideether to give 198 g of 3-cyanomethyl-2-methyl-8-[(3-methyl-2-butenyl)oxy]imidazo[1,2-a]pyridine.

The thus obtained product showed the same physical properties as the compound obtained in Example 16.

The raw material of this example was synthesized as follows.

b)-1 (Chem. Abs., vol. 102, 148704c was referred to)

In 5.5 ℓ of dry ether was dissolved 715 g of methyl levulinate. A catalytic amount of acetic acid-hydrogen bromide was added to the solution. The mixture was cooled to 5°C or lower. To the mixture was dropwise added 283 ml of bromine at an inner temperature between 2 and 5°C (which was carried out while confirming that a red orange color produced by the dropwise addition of bromine disappeared). After completion of the reaction, the reaction mixture was poured onto 6 ℓ of ice water followed by fractionation. After washing with cold saturated sodium hydrogencarbonate aqueous solution, the organic phase was dried

over anhydrous magnesium sulfate. The solvent was distilled (boiling point of 92 to 96°C at about 10 mmHg) to give 642 g (gas chromatography purity: ca. 85%) of methyl 3-bromolevulinate.

b)-2 To 4.3 ℓ of isopropyl alcohol were added 430 g of 2-amino-3-[(3-methyl-2-butenyl)oxy]pyridine and 488 g of triethylamine. Then 870 g (gas chromatography purity: 85 to 90%) of methyl 3-bromolevulinate was added to the mixture followed by stirring to reflux for 2.5 hours. After the solvent was distilled off under reduced pressure, 5 ℓ of water was added to the residue followed by extraction with 6 ℓ of ethyl acetate. After washing with 5 ℓ of water and then with 5 ℓ of saturated sodium chloride aqueous solution, the organic phase was dried over anhydrous magensium sulfate and concentrated to dryness under reduced pressure to give 3-methoxycarbonylmethyl-2-methyl-8-[(3-methyl-2-butenyl)oxy]imidazo[1,2-a]pyridine.

b)-3 The thus obtained compound was dissolved in 2.5 ℓ of methanol. Thereafter ammonia gas was blown into the solution for 10 hours. While ice cooling, 2 ℓ of ether was added to the solution. The formed crystals were taken out by filtration to give 248 g of 3-carbamoylmethyl-2-methyl-8-[(3-methyl-2-butenyl)oxy]-imidazo[1,2-a]pyridine.

Example 39

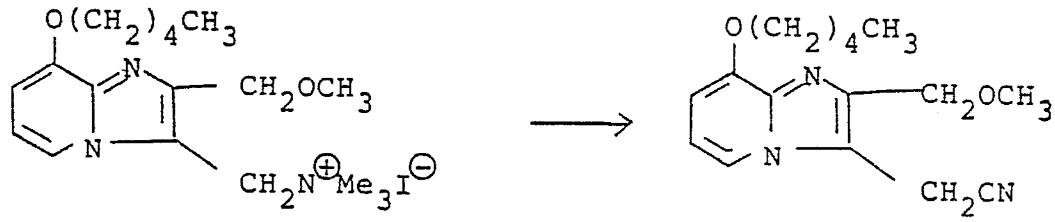

A mixture of 4.0 g (8.94 mmols) of (8-n-pentyloxy-2-methoxymethylimidazo[1,2-a]pyridin-3-yl)methyl trimethylammonium iodide and 0.47 g (9.59 mmols) of sodium cyanide was stirred in 25 ml of dimethylformamide at 99°C for an hour. The reaction solution was carefully poured onto 150 ml of ice water followed by extraction with methylene chloride. After drying over anhydrous magnesium sulfate, the extract was concentrated under reduce pressure. Purification by silica gel column chromatography using chloroform-ethyl acetate as a developing solvent gave 0.7 g (yield 27.3%) of 8-n-pentyloxy-2-methoxymethyl-3-cyanomethylimidazo[1,2-a]pyridine with a melting point of 101 to 102°C.

Elemental analysis (as $C_{16}H_{21}N_3O_2$)

| | C | H | N |
|---|---|---|---|
| Calcd. (%) | 66.88 | 7.37 | 14.62 |
| Found (%) | 66.65 | 7.30 | 14.50 |

Example 40

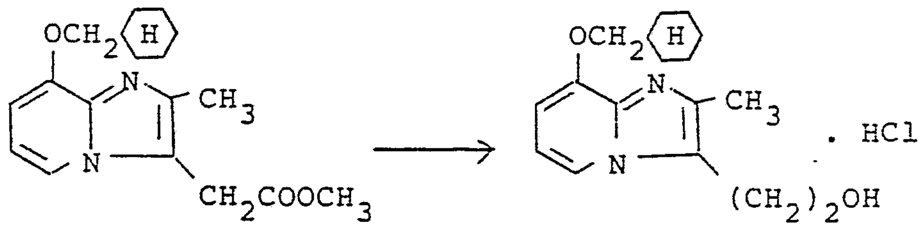

A solution of 0.95 g of 8-cyclohexylmethoxy-2-methyl-3-methoxycarbonylmethylimidazo[1,2-a]pyridine in 5 ml of tetrahydrofuran obtained in a manner similar to Example 38 b)-2 was added to 0.17 g of lithium aluminum hydride in 5 ml of tetrahydrofuran. The mixture was stirred at room temperature for 3 hours. While cooling, 2 ml of ethyl acetate and then saturated sodium sulfate aqueous solution were added to the reaction mixture. Then, ether was added thereto and decantation was performed twice. After the solvent was distilled off under reduced pressure, hydrochloric acid-ethanol was added to make the hydrochloride. Recrystallization from acetonitrile-tetrahydrofuran gave 0.58 g of 8-cyclohexylmethoxy-3-(2-hydroxyethyl)-2-methylimidazo[1,2-a]pyridine hydrochloride.
mp. 147 - 149°C; Mass (EI) m/z 288 ($M^+$)
Elemental analysis (as $C_{17}H_{24}N_2O_2$ HCl $0.1H_2O$)

| | C | H | N | Cl |
|---|---|---|---|---|
| Calcd. (%) | 62.51 | 7.78 | 8.58 | 10.85 |
| Found (%) | 62.45 | 7.80 | 8.36 | 10.71 |

Example 41

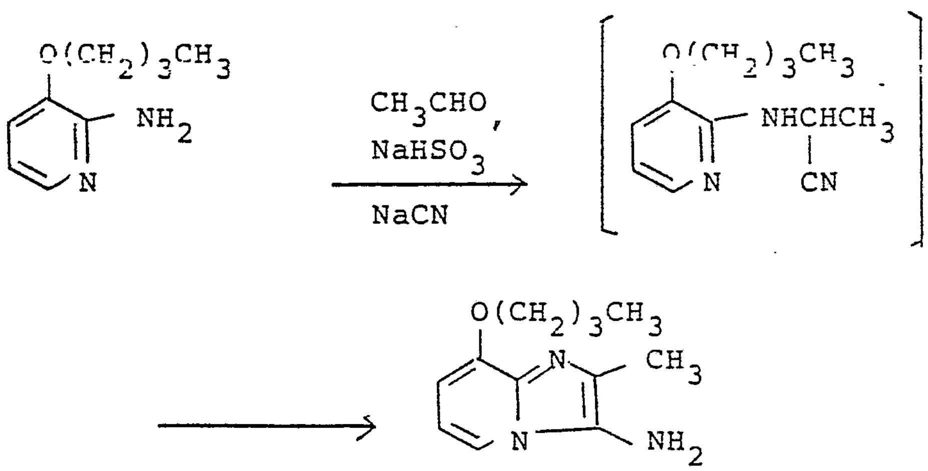


To 5 ml of water was added 2.7 g (18.2 mmols) of acetaldehyde $NaHSO_3$ salt.and 1.0 g (6.0 mmols) of 2-amino-3-n-butyloxypyridine was added to the mixture. After heating to reflux for an hour, the reaction mixture was ice cooled and a solution of 0.95 g (19.3 mmols) of NaCN in 1.6 ml of water was dropwise added thereto. After heating to reflux for 2 hours, 4 ml of methanol was added to the system. After heating to reflux for 6 hours, the reaction mixture was cooled and extracted with chloroform. After washing the extract with water and drying over anhydrous magnesium sulfate, the solvent was distilled off. The residue

was purified by silica gel column chromatography (ethyl acetate) and recrystallized from ethyl acetate to give 0.25 g (19.2%) of 3-amino-8-n-butyl-oxy-2-methylimidazo[1,2-a]pyridine.
mp. 106 - 107°C; Mass (EI) m/z 219 ($M^+$)
Elemental analysis (as $C_{12}H_{17}N_3O$)

| | C | H | N |
|---|---|---|---|
| Calcd. (%) | 65.73 | 7.81 | 19.16 |
| Found (%) | 65.54 | 7.78 | 19.09 |

Example 42

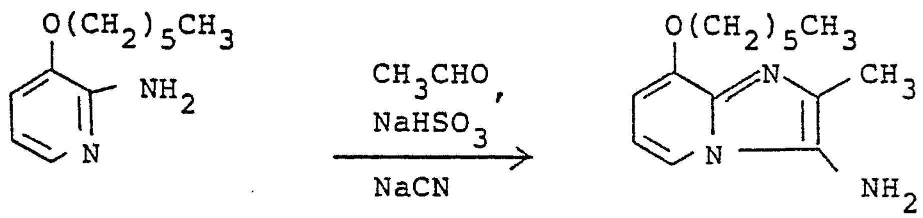


In 1 ml of water was dissolved 0.43 g of sodium hydrogensulfite and, 0.23 ml of acetaldehyde was added to the solution. The mixture was stirred at room temperature for an hour. Then, 0.8 g of 2-amino-3-hexyloxypyridine was added to the mixture at 95°C. After stirring for an hour, an aqueous solution of 0.40 g of sodium cyanide in 0.7 ml of water was added thereto followed by stirring for 2 hours. After cooling, extraction was performed with chloroform. After washing the extract with water, the solvent was distilled off under reduced pressure. The residue was treated by column chromatography and then washed with hexane to give 50 mg of 3-aminop-8-n-hexyloxy-2-methylimidazo[1,2-a]pyridine.
mp. 45 - 65°C; Mass (EI) m/z 247 ($M^+$)
Elemental analysis (as $C_{14}H_{21}N_3O$)

| | C | H | N |
|---|---|---|---|
| Calcd. (%) | 67.98 | 8.56 | 16.99 |
| Found (%) | 67.62 | 8.47 | 16.88 |

Example 43

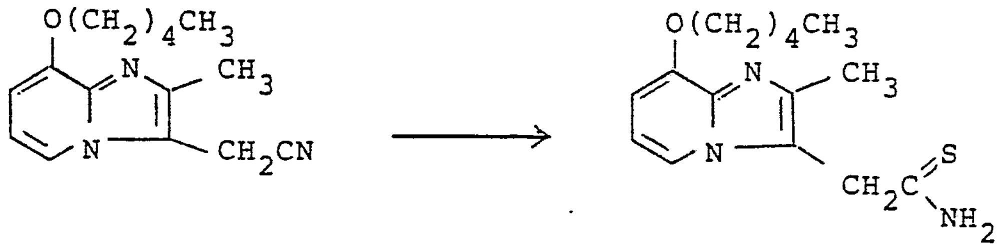

In 50 g of pyridine and 7.85 g of triethylamine was dissolved 20 g (77.72 mmols) of 8-n-pentyl-oxy-2-methyl-3-cyanomethylimidazo[1,2-a]pyridine. Hydrogen sulfide gas was blown into the solution for 4 hours while chilling with water. Then, the reaction mixture was carefully poured onto 300 ml of ice water followed by stirring. The precipitated crystals were taken out by filtration. Recrystallization from ethanol gave 15.11 g (yield, 66.7%) of (8-n-pentyl-oxy-2-methylimidazo[1,2-a]pyridin-3-yl)thioacetamide with a melting point of 157 to 159°C.

Elemental analysis (as $C_{15}H_{21}N_3OS$ 0.1$H_2O$)

| | C | H | N | S |
|---|---|---|---|---|
| Calcd. (%) | 61.44 | 7.29 | 14.33 | 10.94 |
| Found (%) | 61.45 | 7.28 | 14.28 | 10.77 |

Example 44

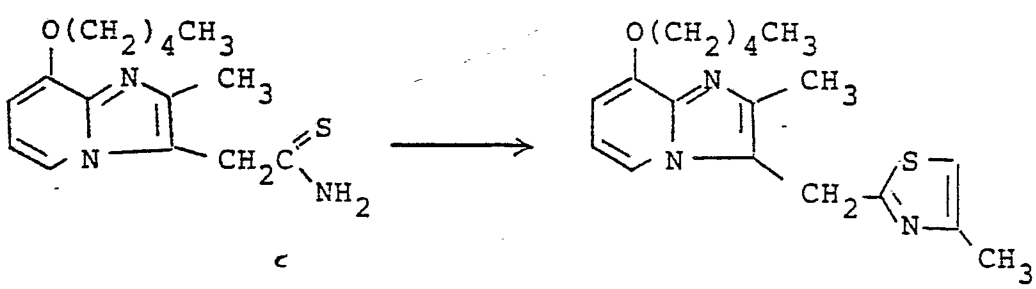

After 1.0 g (3.43 mmols) of (8-n-pentyloxy-2-methylimidazo[1,2-a]pyridin-3-yl)thioacetamide and 0.35 g (3.78 mmols) of chloroacetone were heated in 15 ml of ethanol to reflux for an hour, the solvent was distilled off under reduced pressure. The residue was dissolved in water and potassium carbonate was added to the solution to render the system alkaline. Then extraction was performed with chloroform. The chloroform layer was dried over anhydrous magnesium sulfate and concentrated under reduced pressure. The residue was purified by silica gel column chromatography using a chloroform-ethyl acetate solvent mixture as developing solvent and converted into the fumarate in acetone to give 1.11 g (yield, 73%) of 8-n-pentyloxy-2-methyl-3-(4-methyl-2-thiazolyl)methylimidazo[1,2-a]pyridine fumarate (mp 117 - 118°C).

Elemental analysis (as $C_{18}H_{23}N_3OS.C_4H_4O_4$)

| | C | H | N | S |
|---|---|---|---|---|
| Calcd. (%) | 59.30 | 6.10 | 9.43 | 7.19 |
| Found (%) | 59.15 | 6.09 | 9.34 | 7.16 |

The following compounds were obtained in a similar manner.

Example 45

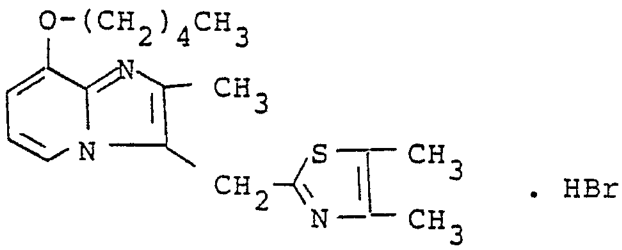

8-n-Pentyloxy-2-methyl-3-(4,5-dimethyl-2-thiazolyl)methylimidazo[1,2-a]pyridine hydrobromide
mp. 166 - 167°C
Elemental analysis (as $C_{19}H_{25}N_3OS.HBr$)

| | C | H | N | S | Br |
|---|---|---|---|---|---|
| Calcd. (%) | 53.77 | 6.17 | 9.90 | 7.56 | 18.83 |
| Found (%) | 53.68 | 6.11 | 9.86 | 7.60 | 18.98 |

Example 46

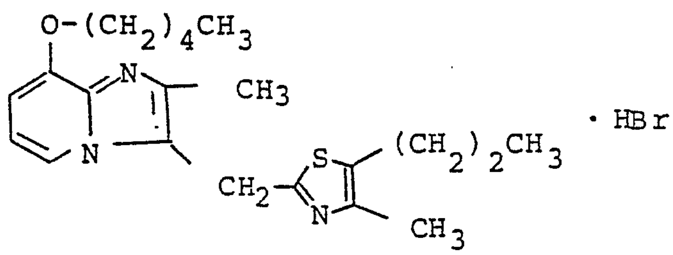

8-n-Pentyloxy-2-methyl-3-(4-methyl-5-n-propyl-2-thiazolyl)methylimidazo[1,2-a]pyridine hydrobromide
mp. 162 - 164°C
Elemental analysis (as $C_{21}H_{29}N_3OS{\bullet}HBr{\bullet}O{\bullet}2H_2O$)

| | C | H | N | S | Br |
|---|---|---|---|---|---|
| Calcd. (%) | 55.30 | 6.71 | 9.21 | 7.03 | 17.52 |
| Found (%) | 55.34 | 6.55 | 9.25 | 7.30 | 17.76 |

Example 47

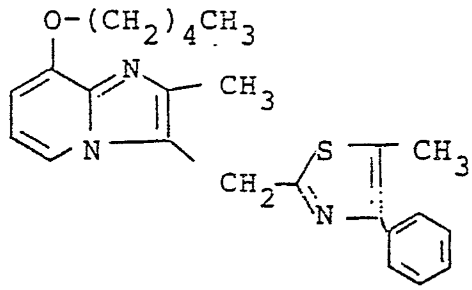

. fumarate

8-n-Pentyloxy-2-methyl-3-(5-methyl-4-phenyl-2-thiazolyl)methylimidazo[1,2-a]pyridine fumarate
mp. 126 - 127°C
Elemental analysis (as $C_{24}H_{27}N_3OS.C_4H_4O_4.\ 0.25H_2O$)

| | C | H | N | S |
|---|---|---|---|---|
| Calcd. (%) | 63.92 | 6.03 | 7.98 | 6.09 |
| Found (%) | 63.75 | 5.90 | 7.77 | 6.05 |

Example 48

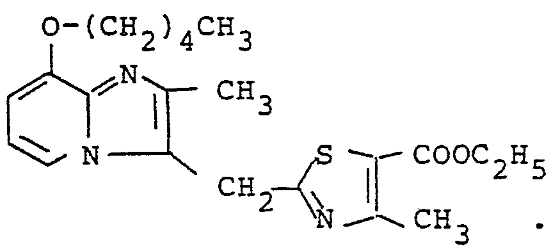

. HCl

8-n-Pentyloxy-2-methyl-3-(5-ethoxycarbonyl-4-methyl-2-thiazolyl)methylimidazo[1,2-a]pyridine hydrochloride
mp. 160 - 163°C
Elemental analysis (as $C_{21}H_{27}N_3O_3S{\bullet}HCl{\bullet}0.4H_2O$)

| | C | H | N | S | Cl |
|---|---|---|---|---|---|
| Calcd. (%) | 56.66 | 6.52 | 9.44 | 7.20 | 7.96 |
| Found (%) | 56.70 | 6.46 | 9.39 | 7.18 | 8.22 |

Example 49

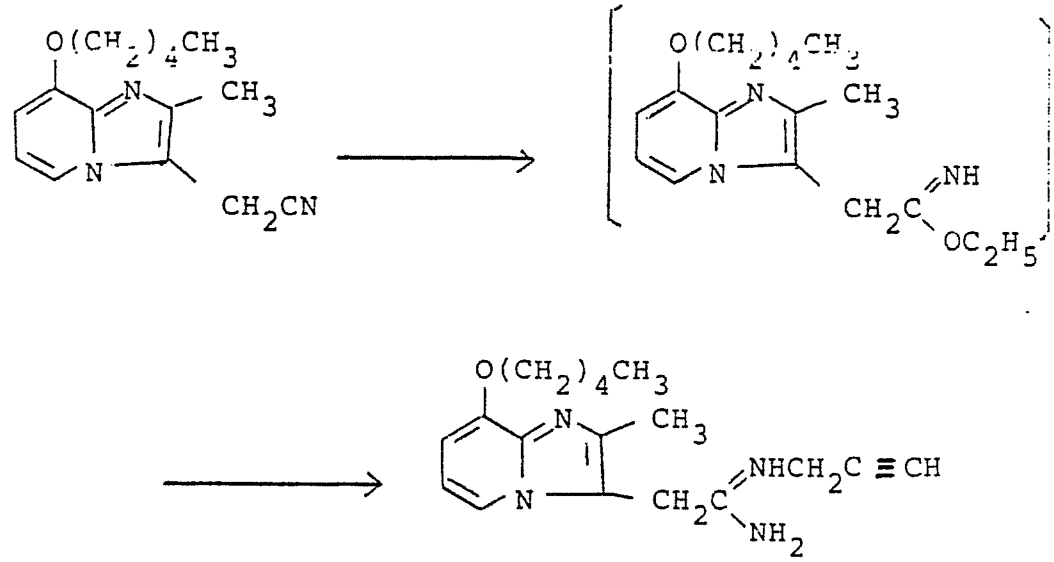

· 3/2 fumarate

a) In a mixture of 5 ml of dry ethanol and 150 ml of dry chloroform was dissolved 10.0 g (38.86 mmols) of 8-n-pentyloxy-2-methyl-3-cyanomethylimidazo[1,2-a]pyridine. The solution was cooled to 0 to 10°C. After hydrogen chloride gas was blown into the solution for 2 hours, the system was allowed to stand for 2 days at about 4°C. The reaction mixture was carefully poured onto ice water in which an excess of potassium carbonate had been dissolved to fractionate the organic phase. The aqueous phase was further extracted with chloroform. Then the extracts were combined with each other followed by drying over anhydrous magnesium sulfate. The solvent was then distilled off under reduced pressure to give 10.1 g (yield, 93%) of oily ethyl (8-n-pentyloxy-2-methylimidazo[1,2-a]pyridin-3-yl)acetimidate.

The imidate was used in the following reaction without purification.

b) After 1.5 g (4.94 mmols) of ethyl (8-n-pentyloxy-2-methylimidazo[1,2-a]pyridin-3-yl)acetimidate and 0.3 g (5.44 mmols) of propargylamine were stirred in 30 ml of ethanol at room temperature for 18 hours, the mixture was reacted 65°C for a further hour. the solvent was distilled off under reduced pressure. The residue was purified by silica gel column chromato graphy using a chloroform-methyl alcohol solvent mixture as a developing solvent and converted into the fumarate in acetone. Thus, 1.1 g of N-propargyl (8-n-pentyloxy-2-methylimidazo[1,2-a]pyridin-3-yl)acetamidine 3/2 fumarate was obtained.

Elemental analysis (as $C_{18}H_{24}N_4O \bullet 3/2\ C_4H_4O_4 \bullet 1/4\ H_2O$)

| | C | H | N |
|---|---|---|---|
| Calcd. (%) | 58.70 | 6.26 | 11.41 |
| Found (%) | 58.69 | 6.37 | 11.32 |

The following compounds were obtained using the amidine of Example 49 a) in a similar manner.

Example 50

N-Sulfamoyl-(8-n-pentyloxy-2-methylimidazo[1,2-a]pyridin-3-yl)acetamidine with a melting point of 194 to 196°C was obtained in a manner similar to Example 49 except for using sulfamide in place of propargylamine.

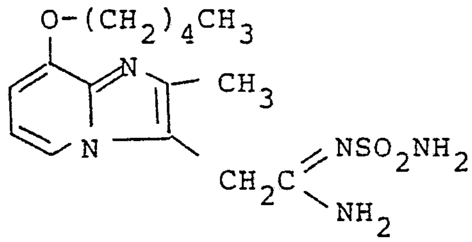

Elemental analysis (as $C_{15}H_{23}N_5O_3S$)

| | C | H | N | S |
|---|---|---|---|---|
| Calcd. (%) | 50.97 | 6.56 | 19.81 | 9.07 |
| Found (%) | 51.07 | 6.61 | 19.23 | 8.90 |

Example 51

N-Acetyl (8-n-pentyloxy-2-methylimidazo[1,2-a]pyridin-3-yl)acetamidorazone having a melting point of 195 to 196°C was obtained in a similar manner except for using acetohydrazide in place of propargylamine.

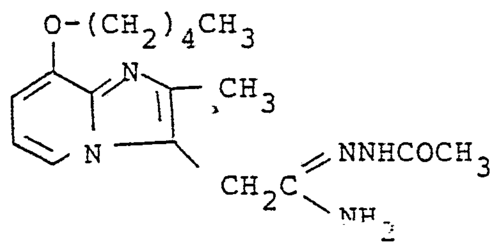

Elemental analysis (as $C_{17}H_{25}N_5O_2$)

| | C | H | N |
|---|---|---|---|
| Calcd. (%) | 61.61 | 7.60 | 21.13 |
| Found (%) | 61.67 | 7.64 | 21.07 |

Example 52

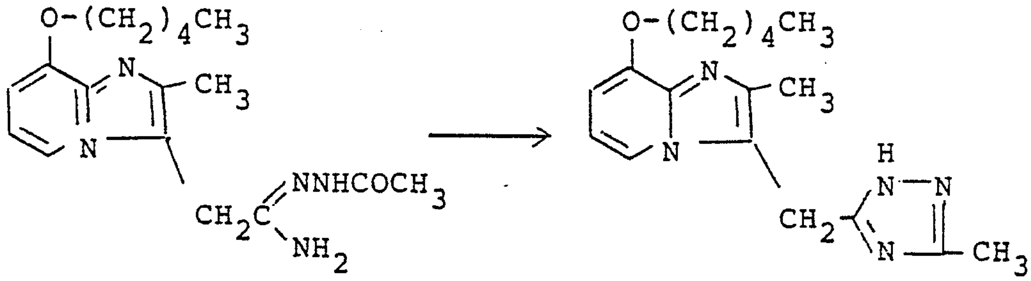

N-Acetyl (8-n-pentyloxy-2-methylimidazo[1,2-a]pyridin-3-yl)acetamidorazone, 9.8 g (2.4 mmols), was heat-treated for 3 minutes at 200°C. Recrystallization from ethanol-ether gave 0.37 g (yield 48.9%) of 3-(8-n-pentyloxy-2-methylimidazo[1,2-a]pyridin-3-yl)methyl-5-methyltriazole with a melting point of 187 to 188°C. Elemental analysis (as $C_{17}H_{23}N_5O$)

| | C | H | N |
|---|---|---|---|
| Calcd. (%) | 65.15 | 7.40 | 22.35 |
| Found (%) | 65.07 | 7.40 | 22.33 |

Example 53

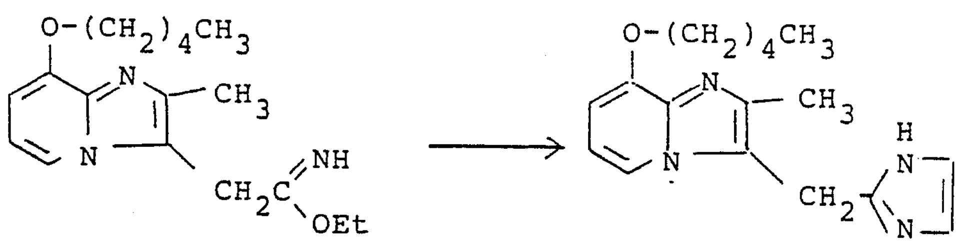

After 1.5 g (4.94 mmols) of ethyl (8-n-pentyloxy-2-methylimidazo[1,2-a]pyridin-3-yl)acetimidate synthesized in Example 49 a) and 0.72 g (5.40 mmols) of aminoacetaldehyde diethyl acetal were stirred at room temperature for 18 hours in 30 ml of ethanol, the mixture was stirred at 65°C for an hour. The solvent was distilled off under reduced pressure and 15 ml of conc. hydrochloric acid was added to the residue. The mixture was stirred at 100°C for 90 minutes and rendered alkaline with a potassium carbonate aqueous solution under ice cooling followed by extraction with chloroform. After drying over anhydrous magnesium sulfate, the chloroform layer was concentrated under reduced pressure. The residue was purified by silica gel column chromatography using a chloroform-methyl alcohol solvent mixture as a developing solvent to give 0.7 g (yield 47.6%) of 8-n-pentyloxy-2-methyl-3-(2-imidazolyl)methylimidazo[1,2-a]pyridine with a melting point of 148 to 149°C.

Elemental analysis (as $C_{17}H_{22}N_4O \cdot 0.2H_2O$)

| | C | H | N |
|---|---|---|---|
| Calcd. (%) | 67.61 | 7.47 | 18.55 |
| Found (%) | 67.97 | 7.49 | 18.16 |

Example 54

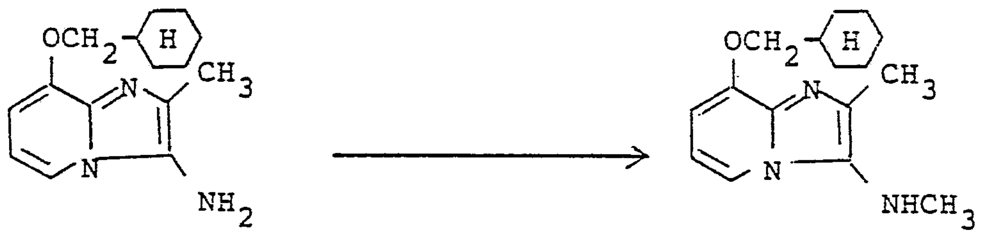

One drop of conc. sulfuric acid was added to a mixture of 1.35 g of 3-amino-8-cyclohexylmethoxy-2-methylimidazo[1,2-a]pyridine and 1.65 g of ethyl orthoformate. The mixture was heated to 150°C over an hour while stirring. After cooling, chloroform was added to the mixture. After washing with a sodium hydrogencarbonate aqueous solution and drying, the solvent was distilled off under reduced pressure. A solution of the residue in 10 ml of tetrahydrofuran was added to a solution of 0.40 g of lithium aluminum hydride in 10 ml of tetrahydrofuran followed by stirring for 1.5 hours under reflux. After cooling, 1 ml of ethyl acetate and then a saturated sodium sulfate aqueous solution were added gently to the reaction mixture. Ether was added to the mixture and decantation was then performed twice. Thereafter the solvent was distilled off under reduced pressure. The residue was treated by column chromatography and then recrystallized from ether-hexane to give 0.31 g of 8-cyclohexylmethoxy-2-methyl-3-methylaminoimidazo[1,2-a]pyridine.

mp. 133 - 135°C; Mass (EI) m/z 273 ($M^+$)

Elemental analysis (as $C_{16}H_{23}N_3O$)

| | C | H | N |
|---|---|---|---|
| Calcd. (%) | 70.30 | 8.48 | 15.37 |
| Found (%) | 70.19 | 8.43 | 15.20 |

Example 55

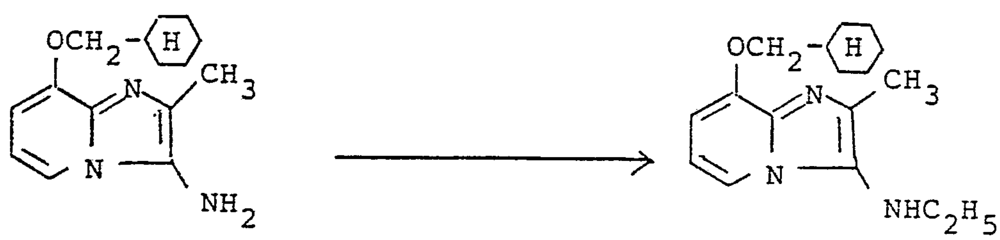

In 10 ml of dry ether were dissolved 0.91 g of 3-amino-8-cyclohexylmethoxy-2-methylimidazo[1,2-a]-pyridine and 0.36 g of triethylamine,and a solution of 0.25 ml of acetyl chloride in 5 ml of ether was dropwise added to the solution at 0°C. After stirring for an hour, chloroform was added to the solution. After washing with water and then drying, the solvent was distilled off under reduced pressure. The residue was recrystallized from ethyl acetate-ether. The thus obtained crystals were added to a tetrahydrofuran solution of 0.14 g of lithium aluminum hydride under argon gas atmosphere followed by stirring under reflux for 1.5 hours. After cooling, 1 ml of ethyl acetate and then saturated sodium sulfate aqueous solution were added gradually to the reaction mixture. After ether was added the mixture, decantation was performed twice. Then, the solvent was distilled off under reduced pressure. After the residue was treated by column chromatography, recrystallization was performed from ether-hexane to give 0.36 g of 8-cyclohexylmethoxy-3-ethylamino-2-methylimidazo[1,2-a]pyridine.

mp. 89 - 80°C; Mass (EI) m/z 287 ($M^+$)

Elemental analysis (as $C_{17}H_{25}N_3O$)

| | C | H | N |
|---|---|---|---|
| Calcd. (%) | 71.05 | 8.77 | 14.62 |
| Found (%) | 71.03 | 8.70 | 14.38 |

Example 56

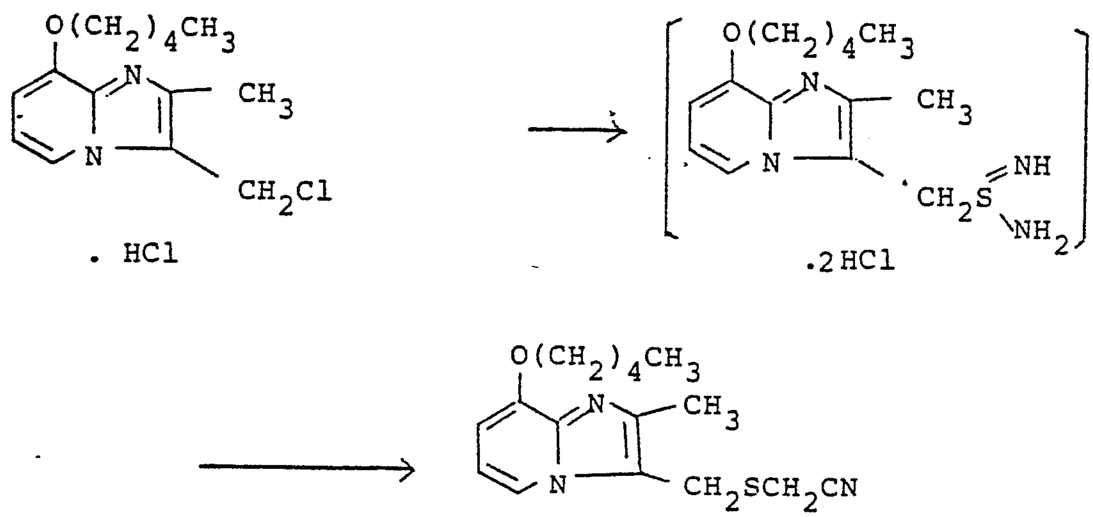

In 50 ml of ethanol was dissolved 1.0 g (3.3 mmols) of 8-n-pentyloxy-3-chloromethyl-2-methylimidazo-[1,2-a]pyridine hydrochloride and 0.25 g (3.3 mmols) of thiourea was added to the solution. After heating to reflux for 2 hours, ethanol was distilled off. The residue was dissolved in 30 ml of ethanol and 30 ml of water and 0.22 ml (3.42 mmols) of chloroacetonitrile was added to the solution. Under ice cooling, an aqueous solution of 0.40 g (10 mmols) of sodium hydroxide in 5 ml of water was dropwise added in argon gas flow. The mixture was stirred for 40 minutes and ethanol was distilled off. Water was added to the residue followed by extraction with ethyl acetate. After drying over anhydrous magnesium sulfate, ethyl acetate was distilled off. The residue was purified by silica gel column chromatography (chloroform : ethyl acetate = 4 : 1) and recrystallized from hexane-ethyl acetate to give 0.30 g (30%) of 3-[cyanomethyl)thio]-methyl-2-methyl-8-n-pentyloxyimidazo[1,2-a]pyridine.
mp. 94 - 95°C; Mass (EI) m/z 303 ($M^+$)
Elemental analysis (as $C_{16}H_{21}N_3OS$)

| | C | H | N | S |
|---|---|---|---|---|
| Calcd. (%) | 63.33 | 6.98 | 13.85 | 10.57 |
| Found (%) | 63.21 | 6.94 | 13.76 | 10.63 |

The following compound was obtained in a similar manner.

Example 57

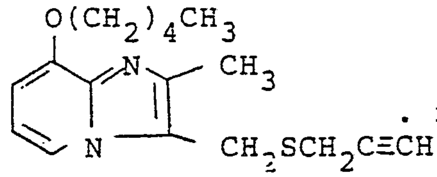

. fumarate

2-Methyl-8-pentyloxy-3-(2-propynylthio)methylimidazo[1,2-a]pyridine fumarate
mp. 127 - 128°C; Mass (EI) m/z 302 ($M^+$)
Elemental analysis (as $C_{17}H_{22}N_2OS.C_4H_4O_4$))

| | C | H | N | S |
|---|---|---|---|---|
| Calcd. (%) | 60.27 | 6.26 | 6.69 | 7.66 |
| Found (%) | 60.32 | 6.35 | 6.65 | 7.76 |

Example 58

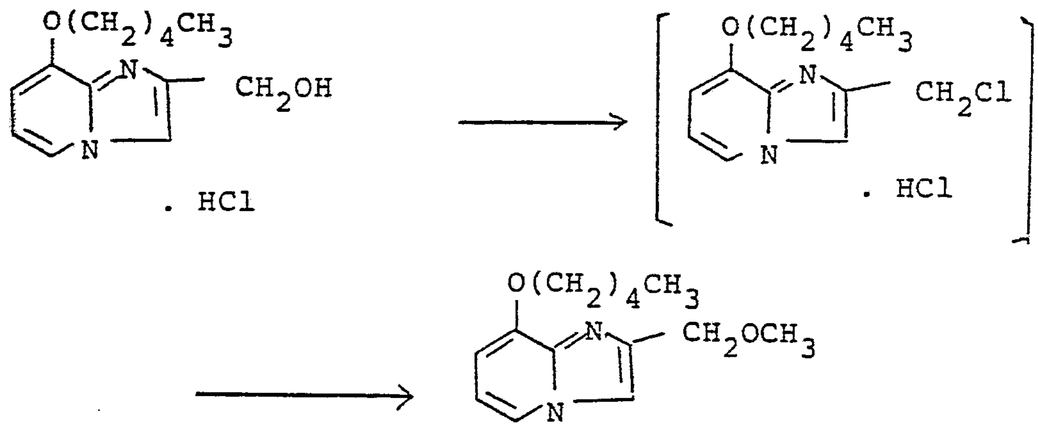

In an argon gas flow, 10.6 g (39.15 mmols) of 8-n-pentyloxy-2-hydroxymethylimidazo[1,2-a]pyridine hydrochloride was added to 70 ml of ice-cooled thionyl chloride. After reverting to room temperature, stirring was continued for 2 hours. Thionyl chloride was distilled off under reduced pressure. The residue was dissolved in 200 ml of methyl alcohol and 5.3 g of sodium methoxide was added to the solution under ice cooling followed by stirring at room temperature for 20 hours. The solvent was distilled off under reduced pressure. The residue was dissolved in ether and the solution was washed with water. After drying over anhydrous magnesium sulfate, the ethereal layer was concentrated under reduced pressure. The precipitated crystals were washed with an ether-n-hexane mixture and filtered to give 7.8 g (yield, 80.4%) of 8-n-pentyloxy-2-methoxymethylimidazo[1,2-a]pyridine with a melting point of 50°C.
Elemental analysis (as $C_{14}H_{20}N_2O_2$)

| | C | H | N |
|---|---|---|---|
| Calcd. (%) | 67.72 | 8.12 | 11.28 |
| Found (%) | 67.67 | 8.05 | 11.21 |

Example 59

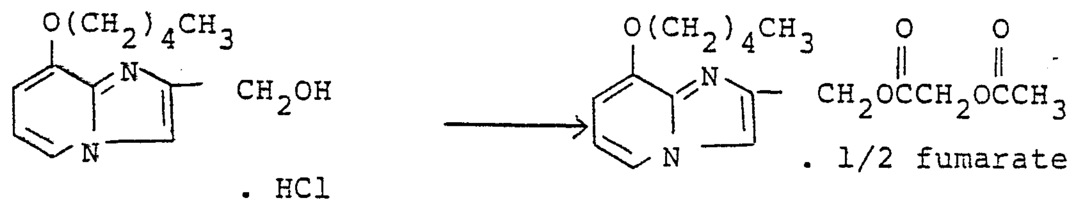

In an argon gas flow, 1.5 g (5.54 mmols) of 8-n-pentyloxy-2-hydroxymethylimidazo[1,2-a]pyridine hydrochloride was suspended in 30 ml of methylene chloride. The suspension was cooled to -20°C on a dry ice-acetone bath. To the suspension was added 1.3 g (12.87 mmols) of triethylamine and, 0.83 g (6.07 mmols) of acetoxyacetyl chloride was further added dropwise thereto. The temperature was gradually reverted to room temperature, at which temperature the mixture was stirred for an hour.

The reaction mixture was washed with water. After drying the methylene chloride layer over anhydrous magnesium sulfate, the solvent was distilled off under reduced pressure. The residue was treated with fumaric acid in ethanol to give 1.2 g (yield, 55.3%) of 8-n-pentyloxy-2-acetoxyacetoxymethylimidazo[1,2-a]pyridine 1/2 fumarate with a melting point of 97°C.

Elemental analysis (as $C_{17}H_{22}N_2O_5$. 1/2 $C_4H_4O_4$))

| | C | H | N |
|---|---|---|---|
| Calcd. (%) | 58.16 | 6.16 | 7.14 |
| Found (%) | 58.06 | 6.12 | 7.13 |

Example 60

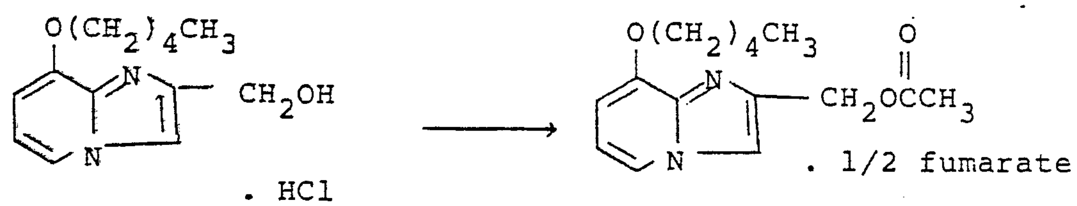

8-n-Pentyloxy-2-acetoxymethylimidazo[1,2-a]pyridine 1/2 fumarate with a melting point of 101 to 102°C was obtained in a manner similar to Example 58 except for using acetyl chloride in place of acetoxyacetyl chloride.

Elemental analysis (as $C_{15}H_{20}N_2O_3$.1/2 $C_4H_4O_4$)

| | C | H | N |
|---|---|---|---|
| Calcd. (%) | 61.07 | 6.63 | 8.38 |
| Found (%) | 60.95 | 6.63 | 8.25 |

Example 61

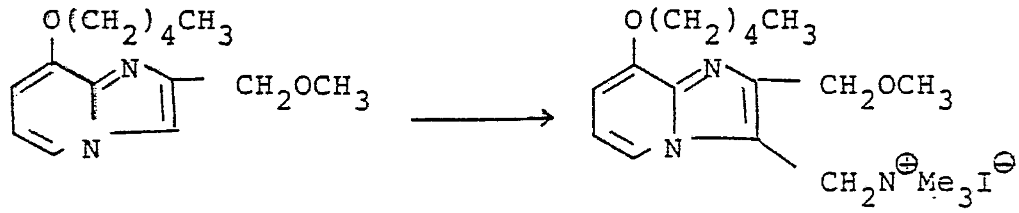

In 70 ml of methyl alcohol, 5.0 g (20.13 mmols) of 8-n-pentyloxy-2-methoxymethylimidazo[1,2-a]-pyridine, 1.8 g (22.07 mmols) of dimethylamine hydrochloride and 0.66 g (21.98 mmols) of paraformaldehyde were heated to reflux for 10 hours. Then, 0.9 g (11.03 mmols) of dimethylamine, hydrochloride and 0.33 g (10.99 mmols) of paraformaldehyde were further added to the reaction mixture followed by heating to reflux for 5 hours. The solvent was distilled off under reduced pressure. The residue was dissolved in water. After washing with ether, the solution was rendered alkaline with potassium carbonate and extracted with ether. After drying the ethereal extract over anhydrous magnesium sulfate, the solvent was distilled off under reduced pressure. The residue was dissolved in 50 ml of ethanol and 3.7 g (26.05 mmols) of methyl iodide was added to the solution. The mixture was stirred at room temperature for 18 hours. The precipitated crystals were filtered to give 7.71 g (yield, 85.7%) of 8-n-pentyloxy-2-methoxymethylimidazo-[1,2-a]pyridin-3-yl)methyl trimethylammonium iodide with a melting point of 155 to 156°C.

Elemental analysis (as $C_{18}H_{30}N_3O_2I \bullet 1/2\ H_2O$)

| | C | H | N |
|---|---|---|---|
| Calcd. (%) | 47.37 | 6.84 | 9.20 |
| Found (%) | 47.58 | 6.63 | 9.02 |

Example 62

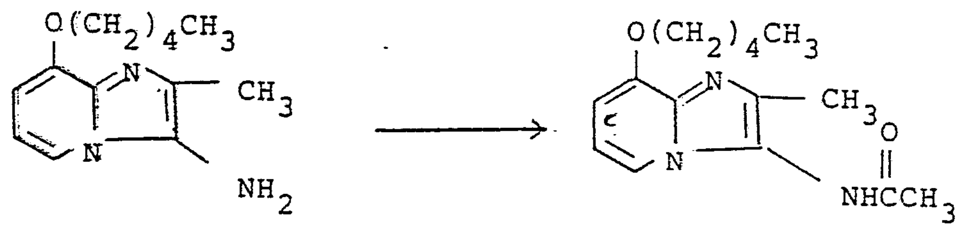

. 1/2 fumarate

In 10 ml of ether was dissolved 1 g of 3-amino-2-methyl-8-n-pentyloxyimidazo[1,2-a]pyridine and 0.43 g of triethylamine was added to the solution. To the solution was added 0.30 ml of acetyl chloride at 5°C or lower. After stirring at room temperature for an hour, the solvent was distilled off under reduced pressure. After treating by column chromatography, the residue was converted into the fumarate. The compound was recrystallized from ethanol-ethyl acetate to give 0.31 g of 3-acetylamino-2-methyl-8-n-pentyloxyimidazo[1,2-a]pyridine•1/2 fumarate.

mp. 183 - 186°C; Mass (EI) m/z 275 ($M^+$)

Elemental analysis (as $C_{15}H_{21}N_3O_2.1/2\ C_4H_4O_4$))

| | C | H | N |
|---|---|---|---|
| Calcd. (%) | 61.25 | 6.95 | 12.60 |
| Found (%) | 60.98 | 6.96 | 12.46 |

Example 63

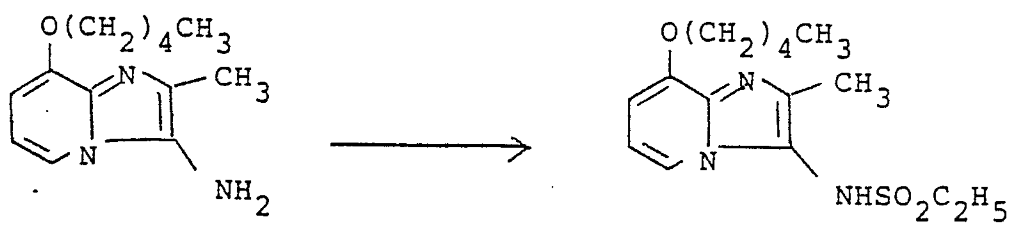

In 10 ml of ether was dissolved 1 g of 3-amino-2-methyl-8-n-pentyloxyimidazo[1,2-a]pyridine and, 0.43 g of triethylamine was added to the solution. To the solution was added 0.4 ml of ethyl sulfonyl chloride at 5°C or lower. The mixture was stirred at room temperature for an hour. Ethyl acetate was added thereto followed by washing with water. Then, the solvent was distilled off under reduced pressure. After heating to reflux in saturated hydrogen chloride-ethanol, the solvent was distilled off under reduced pressure. The residue was recrystallized from ethyl acetate-isopropyl ether to give 0.13 g of 3-ethylsufonylamino-2-methyl-8-n-pentyloxyimidazo[1,2-a]pyridine.
mp. 114 - 115°C; Mass (EI) m/z 325 ($M^+$)
Elemental analysis (as $C_{15}H_{23}N_3O_3S$)

| | C | H | N | S |
|---|---|---|---|---|
| Calcd. (%) | 55.36 | 7.12 | 12.91 | 9.85 |
| Found (%) | 55.57 | 6.96 | 12.80 | 9.83 |

**Claims**

1. An imidazopyridine derivative represented by general formula (I) or a salt thereof:

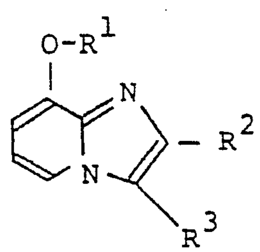

(I)

wherein $R^1$ represents an alkenyl group having 4 to 6 carbon atoms or an alkyl group having 1 to 10 carbon atoms which may have a cycloalkyl group substituent; $R^2$ represents a hydroxy group, a ($C_1$-$C_6$ alkoxy)-carbonyl group, a $C_1$-$C_6$ alky group which may have a hydroxy, $C_1$-$C_6$ alkoxy or ($C_1$-$C_6$ alkoxy)carbonyl group substituent, a phenyl group which may have a $C_1$-$C_6$ alkoxy group substituent, or a ($C_1$-$C_6$ acyloxy)alkyl group which have a $C_2$-$C_6$ acyloxy group substituent : and $R^3$ represents a hydrogen atom, a $C_1$-$C_6$ alkyl group, a halogen-($C_1$-$C_6$ alkyl) group, a ($C_1$-$C_6$ alkoxy)alkyl group, a hydroxy-($C_1$-$C_6$ alkyl) group, a cyano ($C_1$-

$C_6$ alkyl) group, a mono-or di-($C_1$-$C_6$ alkyl)amino group, a nitroso group, a ($C_1$-$C_6$ alkoxy)carbonyl group, a $C_2$-$C_6$ acylamino group, a $C_1$-$C_6$ alkylsulfonylamino group, a group of formula:

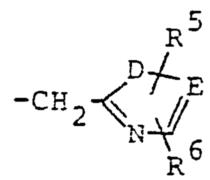

(wherein D is NH or S, E is N or CH, and $R^5$ and $R^6$ are the same or different and selected from a hydrogen atom and $C_1$-$C_6$ alkyl, ($C_1$-$C_6$ alkoxy)carbonyl and phenyl groups); a group of formula:

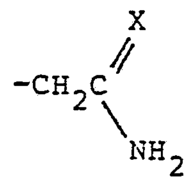

(wherein X is O, S or N-$R^7$ wherein $R^7$ is a sulfamoyl group, a $C_2$-$C_6$ acylamino group or a $C_2$-$C_6$ alkynyl group); or a group of formula:-

$-CH_2$-Y-$R^8$

(wherein Y is O is S and $R^8$ is a cyano-($C_1$-$C_6$ alkyl) group or a $C_2$-$C_6$ alkynyl group).

2. A compound according to claim 1 wherein $R^2$ is a $C_1$-$C_6$ alkyl group which may have a hydroxy, $C_1$-$C_6$ alkoxy)carbonyl group substituent or a ($C_2$-$C_6$ acyl)oxyalkyl group which may have a $C_2$-$C_6$ acyloxy group substituent; and $R^3$ is a $C_1$-$C_6$ alkyl group, a halogeno-($C_1$-$C_6$ alkyl) group, a ($C_1$-$C_6$ alkoxy)alkyl group, a hydroxy-($C_1$-$C_6$ alkyl) group, a cyano-($C_1$-$C_6$ alkyl) group, an amino group, a mono-or di-($C_1$-$C_6$ alkyl)amino group, a nitroso group or a ($C_1$-$C_6$ alkoxy)carbonyl group.

3. A compound according to claim 1 wherein $R^2$ is a $C_1$-$C_6$ alkyl group and $R^3$ is a cyano-($C_1$-$C_6$ alkyl) group.

4. A compound according to claim 1 selected from
3-cyanomethyl-2-methyl-8[(3-methyl-2-butenyl)-oxy]imidazo[1,2-a]pyridine;
3-cyanomethyl-8-cyclohexylmethoxy-2-methyl-imidazo[1,2-a]pyridine; 3-cyanomethyl-2-methyl-8-pentyloxyimidazo[1,2-a]pyridine; and salts thereof.

5. A process (a) for production of imidazopyridine derivative of formula (Ia) or pharmaceutically acceptable salt thereof

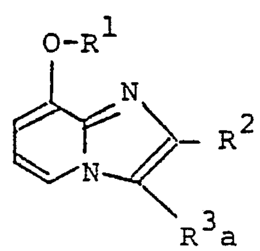

(Ia)

which process comprises reacting amino-substituted pyridyl ether of formula (II)

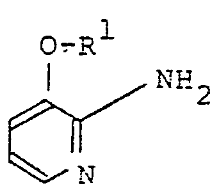

(II)

with compound of formula (III)

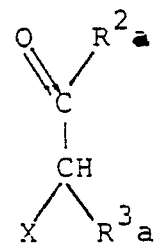

(III)

wherein $R^1$ and $R^2$ are as defined in claim 1; $R^2a$ represents a halogen atom, a ($C_1$-$C_6$ alkoxy)carbonyl group, a $C_1$-$C_6$ alkyl group which may have a ($C_1$-$C_6$ alkoxy)carbonyl group substituent or a phenyl group which have with a $C_1$-$C_6$ alkoxy group substituent; $R^3a$ represents a hydrogen atom, a $C_1$-$C_6$ alkyl group, a cyano-($C_1$-$C_6$ alkyl) group or a ($C_1$-$C_6$ alkoxy)carbonyl group; and X represents a halogen atom or an organic sulfonic acid residue; and optionally converting the product to or from salt form; or (b) for production of imidazopyridine derivative of formula (Ih) or pharmaceutically acceptable salt thereof

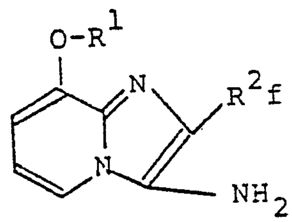

(Ih)

which process comprises reacting amino-substituted pyridine ether of formula (II)

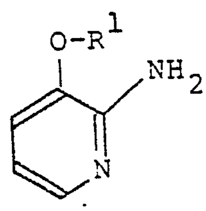

(II)

with aldehyde of formula (VII)
$R^2f$-CHO (VII)
and alkali metal cyanide of formula (VIII)
MCN (VIII)
wherein $R^1$ is as defined in claim 1; $R^2f$ is a $C_1$-$C_6$ alkyl group; and wherein M represents an alkali metal atom; and optionally converting the product to or from salt form.

6. A process for production of imidazopyridine derivative (a) of formula (Ib) or (Ib') or pharmaceutically acceptable salt thereof

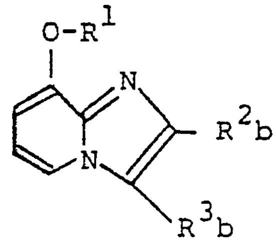

or

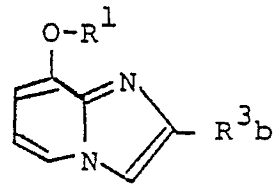

(Ib) (I b')

the process comprising reducing compound of formula (IV) or (IV')

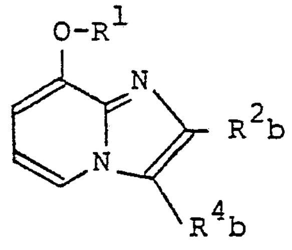

or

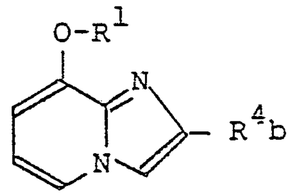

(IV) (IV')

wherein $R^1$ is as defined in claim 1; $R^2b$ is a $C_1$-$C_6$ alkyl group; $R^3b$ is a hydroxy-($C_1$-$C_6$ alkyl) group; a cyano-($C_1$-$C_6$ alkyl) group or an amino group and $R^4b$ is a group capable of being reduced to a hydroxy-($C_1$-$C_6$ alkyl) group or an amino group; or (b) of formula (Id) or pharmaceutically acceptable salt thereof

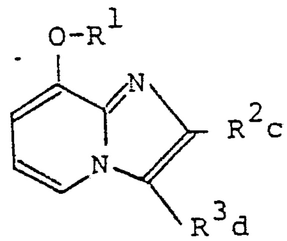

(Id).

the process comprising reacting compound of formula (Ic)

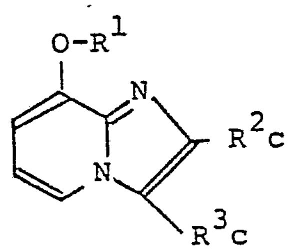

(Ic)

with halogenating agent, wherein $R^1$ is as defined in claim 1; $R^2c$ is a $C_1$-$C_6$ alkyl group or a phenyl group which may have a $C_1$-$C_6$ alkoxy group substituent; $R^3d$ is a halogeno-($C_1$-$C_6$ alkyl) group and $R^3c$ is a hydroxy-($C_1$-$C_6$ alkyl) group; or (c) of formula (Ie) or pharmaceutically acceptable salt thereof

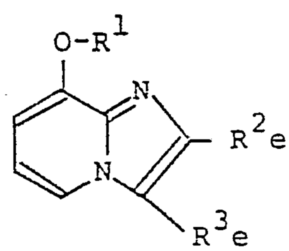

(Ie)

the process comprising reacting compound of formula (V)

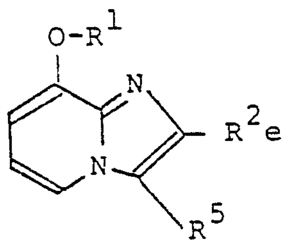

(V)

with cyanogenating agent or dehydrating agent wherein $R^1$ is as defined in claim 1; $R^2e$ is a $C_1$-$C_6$ alkyl group which may be substituted by a $C_1$-$C_6$ alkoxy group, or a phenyl group which may have a $C_1$-$C_6$ alkoxy group substituent; $R^3e$ is a cyano-($C_1$-$C_6$ alkyl) group; and $R^5$ is a group capable of being converted into a cyano-($C_1$-$C_6$ alkyl) group; or (d) of formula (Ig) or pharmaceutically acceptable salt thereof

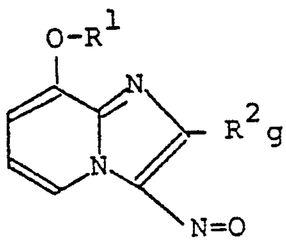

(Ig)

the process comprising reacting compound of formula (If)

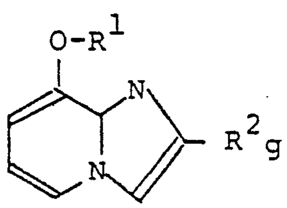

(If)

with nitroso agent of formula (VI)

$R^9ONO$ (VI)

wherein $R^1$ is as defined in claim 1; $R^2g$ is a $C_1$-$C_6$ alkyl group or a phenyl group which may have a $C_1$-$C_6$ alkoxy group substituent; and $R^9$ is an alkali metal atom, a hydrogen atom or a $C_1$-$C_6$ alkyl group; each of processes (a) to (d) optionally including converting the product to or from salt form.

7. A process of producing a compound according to claim 1 which comprises performing a process according to claim 5 or 6 and further converting a substitutent on the product, optionally followed by conversion to or from salt form.

8. A pharmaceutical composition comprising a pharmacologically effective amount of imidazopyridine derivative or pharmaceutically acceptable salt according to any one of claims 1 to 4 and a pharmaceutically acceptable carrier.

9. A compound of formula

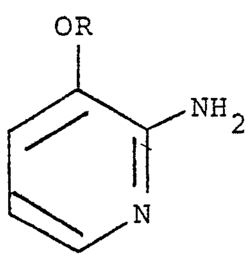

wherein R is ethyl, propyl, butyl, pentyl, hexyl, heptyl, octyl, 2-methyl-propyl, 1-methyl-butyl, 2-ethyl-butyl, butenyl, 3-methyl-butenyl, methyl-cyclohexyl or ethyl-cyclohexyl.

European Patent Office

# EUROPEAN SEARCH REPORT

Application Number

EP 87 30 8663

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | TETRAHEDRON LETTERS, vol. 26, no. 21, 1985, pages 2571-2574, Pergamon Press Ltd, Oxford, GB; R. RYDZKOWSKI et al.: "Synthesis of new heterocyclic phenols: 8-hydroxy-imidazo [1,2-a] pyridine" * Page 2573, compound 4 * --- | 1 | C 07 D 471/04<br>A 61 K 31/435<br>C 07 D 213/76 //<br>(C 07 D 471/04<br>C 07 D 235:00<br>C 07 D 221:00 ) |
| D,X | EP-A-0 068 378 (SCHERING) * Claim 1; page 44, lines 12-22; page 45, lines 1-4 * --- | 1,8 | |
| X | EP-A-0 120 589 (FUJISAWA) * Page 51, example 20, lines 21,22 * ----- | 9 | |
| | | | TECHNICAL FIELDS SEARCHED (Int. Cl.4) |
| | | | C 07 D 471/00<br>A 61 K 31/00<br>C 07 D 213/00 |

The present search report has been drawn up for all claims

| Place of search | Date of completion of the search | Examiner |
|---|---|---|
| THE HAGUE | 13-01-1988 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document